# EUROPEAN PATENT APPLICATION

(11) **EP 4 495 108 A1**
(43) Date of publication of application: **22.01.2025**
(21) Application number: 23185725.1
(22) Date of filing: 17.07.2023
(51) Int. Cl.: C07D 317/34, C08J 5/24, C08K 5/1565, C09J 11/06

(54) **3-[2-(2-OXO-1,3-DIOXOLAN-4-YLIDENE)ETHOXY]PROPANOIC ACID DERIVATIVES AS PRECURSORS OF MONOMERS IN POLYMERISATIONS**

(71) Applicant: BASF SE, 67056 Ludwigshafen am Rhein (DE); Albert-Ludwigs-Universität Freiburg, 79098 Freiburg (DE)
(72) Inventor: RUDOLF, Peter, 67056 Ludwigshafen am Rhein (DE); FUKUZUMI, Takeo, 67056 Ludwigshafen am Rhein (DE); GANSCHOW, Michael, 79104 Freiburg (DE); MERKENS, Kay, 79110 Freiburg (DE); RUEHE, Juergen, 79110 Freiburg (DE)
(74) Representative: BASF IP Association

(57) **Abstract**

The present invention relates to functionalized cyclic carbonates with an exocyclic vinylidene group of formula (I) 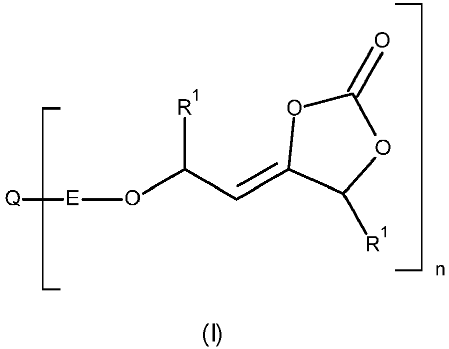 a process for the production of these functionalized cyclic carbonates of formula (I) in the present of a transition metal catalyst and the use of the functionalized cyclic carbonates as monomers in polymer applications.

## Description

The present invention relates to functionalized cyclic carbonates with an exocyclic vinylidene group of formula (I) 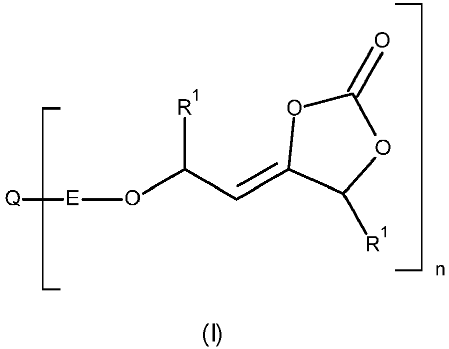 a process for the production of these functionalized cyclic carbonates of formula (I) in the present of a transition metal catalyst and the use of the functionalized cyclic carbonates as monomers in polymer applications.

Exo-vinylene carbonates are valuable compounds, especially as monomers in polymer applications as described in WO 2011/157671 A1.

The exo-vinylene carbonates mentioned in WO 2011/157671 were used as reactive diluent in epoxide resins and are all based on propargyl derivates. During the curing process of the epoxide the exo-vinylene carbonate reacts with the epoxide /amin formulation and does not leave the epoxide resin. Thus, these special exo-vinylene carbonates based on propargyl alcohol described in WO 2011/157671 react as chain termination agents.

There is a high demand of exo-vinylene carbonates which act as active monomers or as precursors in polymerization reactions. In order to be an active part in a polymerization reaction the exo-vinylene carbonates need at least one further functional group beside the exo-vinylene group, exo-vinylene carbonates based on propargyl alcohols don't have a further functional group which can be used for the polymerization.
Therefore, the precursor compounds of the exo-vinylene carbonates need at least two functional groups, one to form the exo-vinylene group and another one for enabling the polymerization reaction or at least two exo-vinylene functional groups.

Instead of propargyl alcohol dialcohols like 1 ,4-butyndiole or 2,5-hexyndiol might be better starting products for the precursor compounds of the exo-vinylene carbonates. But there are different risks mentioned in the prior art which seems to teach away from using dialcohols like 1,4-butyndiole or 2,5-hexyndiol.

US 4,487,781, WO 2020/113233, WO 2017/76998 A1 and Tv. Golobokova et al. in Zh. Org. Khim., 2013 vol 49, No. 1, p. 135-141 describe the oxo-Michael-addition reaction between propargyl alcohol with an acrylic acid ester for different steps in the synthesis of active components. The Oxo-Michael addition between one OH-group of 1,4-butyndiol or 2,5-hexyndiol with an α,β -functional group in order to get a precursor for an exo-vinylene carbonates with a further functional group is not described in these documents.

T.A. Favorskaya and S.A. Poroshina et al. describe in Zhurnal Organicbeskoi Khimii, Vol.6, No. 7, p 1416-1419, July 1970 the oxa-Michael-Addition of 1,4-butyndiol with methyl acrylate or acrylonitrile. They describe that they use two mols of methyl acrylate with one mol of 1,4-butyndiol and isolate 24% of 2-(methoxycarbonyl) ethyl ether of butyn1,4-diol (the mono ester) via distillation and also receive 40% of the bis[2-(methoxycarbonyl)ethyl] ether of butyn-1,4-diol (the diester). The use of such mono esters for the preparation of exo-cyclic carbonates are not mentioned here.

Daniel Hennen et al. in ChemPhotoChem 2020, 4, 476 - 480 describe the oxa-Michael addition reaction between butandiacrylate and propargyl alcohol. The received main product is BBP, but there are further by-products which are caused by transesterification which is a big challenge during the oxa-Michael addition of alcohols to a diacrylate derivate. The selective oxa-Michael addition to receive most of the main product without or limited amount of transesterification products is not disclosed.

Karin Ratzenböck et al. describes in Poly. Chem 11, 7476 also the problem of transesterification during polymerisation of dialcohols with ethylacrylate.

WO 2019/034648 A1 and WO 2021/144162 A1 describe the production of exo vinylene carbonates by using propargylic alcohols with CO₂ in the presence of special transition metal catalyst, preferable Ag, with at least one bulky ligand, particular a phosphor ligand. The definition of "propargylic alcohols" mentioned in WO 2019/034648 is not restricted to prop-2-yn-1-ol. It also means all compounds that comprises the prop-2-yn-1-ol group. Examples where 1,4-butynediol is reacted with a halogen compound via a substitution reaction to the corresponding alkynyl ether compound are disclosed in WO 2019/034648. The reaction of the Michael oxo addition products of 1,4-butynediol and/or 2,5-hexyndiol with an α,β -functional groups according to formula (II) and the reaction of these alkynyl ether compounds to an exocyclic carbonate as mentioned in formula (I) are not disclosed either in WO 2019/034648 nor in WO 2021/144162.

WO 2019/219469 describes the production process of exo vinylene carbonates starting form a 4-oxy-but-2-yn-1-ol derivative in the presence of CO₂ and a transition metal catalyst comprising Ag as central atom and a bulky ligand. WO 2019/219469 describes that theses exo vinylene carbonates can be used as monomers in polymerization reactions or in oligomerization reactions. The 4-oxy-but-2-yn-1-ol derivates described in WO 2019/219469 as well as the resulting exo vinylene carbonates are such that carry preferably no or only one methylene group as Z group between the 4-oxy group and the following carbonyl group (-C(O)-) of the 4-oxy-but-2yn-1-ol derivate so that urethanes, carbonates and ester compounds result. It was now found that these urethanes, carbonates and ester compounds will decompose during polymerization reactions and CO₂ will be split off. WO 2019/219469 does not describe that at least an ethylene group has to be located between the 4-oxy- and the carbonyl (-C(O)-) group of the 4-oxy-but-2-yn-1-ol derivate in order to receive functional exocyclic carbonates that are able to be used as valuable precursor of monomers in polymerization reactions.

Therefore, it is an object to the present invention to provide new functionalized cyclic carbonates with an exocyclic vinylidene group which are able to be used as precursor of monomers or active monomers in polymerization reactions and which open a new field of polymers where such cyclic carbonates form a new group of polymers and influences the properties of the polymers.

This problem will be solved with functionalized cyclic carbonates with an exocyclic vinylidene group of formula (I) 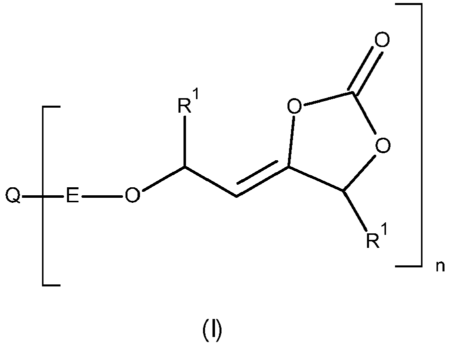 with
- n: is a natural number selected from the 1 to 10,
- R¹: is selected from the group of H and methyl,
- E: is selected from the group of -CH₂-CH₂-, -CH₂-CH₂-C(O)-, and 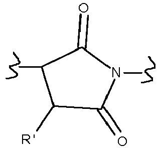 with R' is selected from the group of H and a C₁-C₈ alkyl group, wherein the carbon atom of the terminal -CH₂- or the -CH- group next to the -CHR'-group of E is bonded to the -O- of formula (I)
- Q: is dependent on n and E
for n=1 and E= -CH₂-CH₂-
   Q is selected from the group of -CN and substituted or unsubstituted 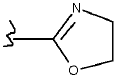 (2-oxazolin) group,
for n=1 and E= -CH₂-CH₂-C(O)-
   Q is selected from the group of -O-Y, -NR²-Y and 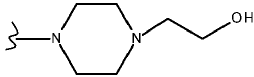 (4-(2-hydroxyethyl)piperazin-1-yl group ,
for n=1 and E= 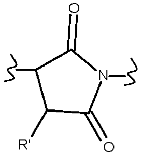 Q is selected from the group of substituted or unsubstituted C₆-C₄₀ aryl groups,
for n=2 and E= -CH₂-CH₂-
   Q is selected from the group of a divalent bonded sulfone (-S(O)₂-) group, a divalent substituted or unsubstituted 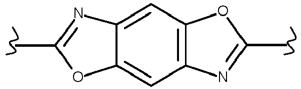 group and a divalent substituted or unsubstituted 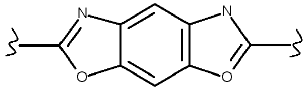 (benzobisoxazol-2,6-diyl) group,
for n=2 and E= -CH₂-CH₂-C(O)-
   Q is a divalent -A- group,
for n=2 and E= 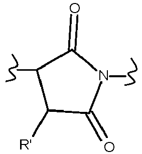 Q is selected from the group of divalently bonded substituted or unsubstituted C₄-C₁₀ alkylen groups, divalently bonded substituted or unsubstituted phenyl, divalently bonded substituted or unsubstituted C₁₃-C₄₀ arylalkylenaryl groups and divalently bonded substituted or unsubstituted C₈-C₄₀ alkylenarylalkylen groups,
for n=3 and E= -CH₂-CH₂-C(O)-
   Q is selected from the group of a substituted or unsubstituted 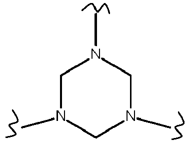 (1,3,5-triazinan) group, 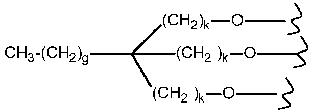 group, 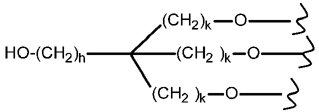 group and 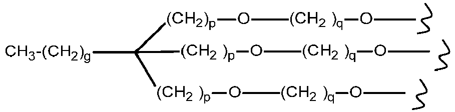 group wherein
   g is a natural number selected from 1 to 33,
   k is a natural number selected from 1 to 12,
   h is a natural number selected from 1 to 15,
   p is a natural number selected from 1 to 3 and
   q is a natural number selected from 1 to 5,
for n≥4 and E= -CH₂-CH₂-C(O)-
   Q is selected from the group of 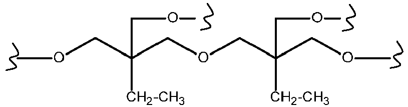 and 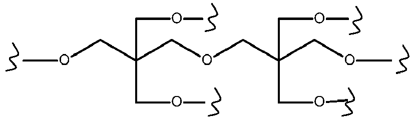 and
   with R² selected from the group of H, substituted or unsubstituted C₁-C₄₀ alkyl groups, substituted or unsubstituted C₃-C₄₀ cycloalkyl groups, substituted or unsubstituted saturated or unsaturated heterocyclic groups containing 2 to 20 carbon atoms and one or more heteroatoms selected from the group of N, O, and S as ring members, substituted or unsubstituted C₆-C₄₀ aryl groups, substituted or unsubstituted C₂-C₄₀-alkenyl groups, C₂-C₄₀ alkynyl groups, substituted or unsubstituted C₄-C₄₀ alkylencycloalkyl groups and substituted or unsubstituted C₁-C₄- alkylen groups to be a part of a substituted or unsubstituted saturated or unsaturated heterocyclic group that is built up between R² of the -NR²- group and Y which is not H, to form 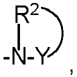 with Y is selected from the group of H, substituted or unsubstituted C₁-C₄₀ alkyl groups, substituted or unsubstituted saturated or unsaturated C₃-C₄₀ cycloalkyl groups, substituted or unsubstituted C₂-C₄₀ (poly)ether groups, substituted or unsubstituted saturated or unsaturated heterocyclic groups containing 2 to 20 carbon atoms and one or more heteroatoms selected from the group of N, O, and S as ring members, substituted or unsubstituted saturated or unsaturated alkylenheterocyclic groups containing 3 to 20 carbon atoms and one or more heteroatoms selected from the group of N, O, and S as ring members, substituted or unsubstituted C₆-C₄₀ aryl groups, substituted or unsubstituted C₂-C₄₀ alkenyl groups, substituted or unsubstituted C₂-C₄₀ alkynyl groups, substituted or unsubstituted saturated or unsaturated C₃-C₄₀ alkylencycloalkyl groups, substituted or unsubstituted C₇-C₄₀ alkylenaryl groups, substituted or unsubstituted α,β- unsaturated carbonyl groups containing C₃-C₄₀ atoms, substituted or unsubstituted C₂-C₅ keto groups, and substituted or unsubstituted C₁-C₄ alkylen group which is part of a substituted or unsubstituted saturated heterocyclic group that is built up between R² of the -NR²- group and Y which is not H to form 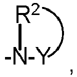 with -A- is one of the following bridging groups:

   -X¹-Z-X²-

   or 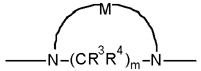 wherein X¹, X² are independently selected from -O-, and -NR²- and
   Z is selected from the group of substituted or unsubstituted C₁-C₄₀ alkylen groups, divalently substituted or unsubstituted C₂-C₄₀ (poly)ether groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded substituted or unsubstituted alkylenheterocyclicalkylen groups containing 4 to 20 carbon atoms and one or more heteroatoms selected from the group of N, O, and S as ring members, divalently bonded substituted or unsubstituted C₆-C₄₀ aryl groups, divalently bonded substituted or unsubstituted C₇-C₄₀ cycloalkylalkylencycloalkyl groups, divalently bonded substituted or unsubstituted saturated or unsaturated C₅-C-₄₀ alkylencycloalkylalkylen groups, divalently bonded substituted or unsubstituted C₁₃-C₄₀ arylalkylenaryl groups, divalently bonded substituted or unsubstituted C₈-C₄₀ alkylenarylalkylen groups and divalently bonded substituted or unsubstituted C₁₇-C₃₀ alkylen-bisphenol A -alkylen groups and
   R³ and R⁴ are independently selected from the group of H and substituted or unsubstituted C₁-C₄₀ alkyl groups,
   m is a natural number selected from the group of 1, 2 or 3 and
   M is selected from the group of substituted or unsubstituted C₁-C₃ alkylen groups, divalently bonded -NR⁵-CR³R⁴- groups, divalently bonded -NR⁵-NR⁶- groups, divalently bonded-NR⁵-CR³R⁴-CR³R⁴- groups, divalently bonded -O-CR³R⁴-CR³R⁴- groups, divalently bonded -S-CR³R⁴-CR³R⁴- groups, divalently bonded CR³R⁴-O-CR³R⁴ groups, divalently bonded -CR³R⁴-S-CR³R⁴- groups and divalently bonded -CR³R⁴-NR⁵-CR³R⁴- groups
   with R⁵ and R⁶ are independently selected from the group of H, substituted or unsubstituted C₁-C₄₀ alkyl groups, C₆-C₄₀ aryl groups, C₃-C₄₀ cycloalkyl groups and -C(O)CH=CH₂ group.

The inventive functionalized cyclic carbonates will be advantageous, wherein E = -CH₂-CH₂- , n is selected from the group of 1 and 2 and Q is selected from the group of -CN, with C₁-C₄ alkylgroups or C₁-C₄ alkylhydroxy groups substituted 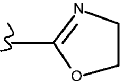 (2-oxazolin) groups, a divalent bonded sulfone (-S(O)₂-), a divalent substituted or unsubstituted 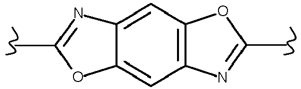 group and a divalent substituted or unsubstituted 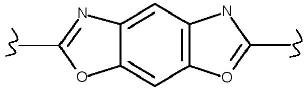 (benzobisoxazol-2,6-diyl) group.

The inventive functionalized cyclic carbonates will be advantageous, wherein E = 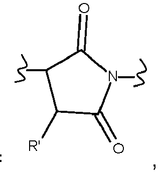 n is selected from the group of 1 and 2 and Q is selected from the group of phenyl, substituted or unsubstituted divalently bonded C₆-C₄₀ aryl groups, divalently bonded substituted or unsubstituted C₁₃-C₄₀ arylalkylenaryl groups, divalently bonded substituted or unsubstituted C₈-C₄₀ alkylenarylalkylen groups.

The inventive functionalized cyclic carbonates will be advantageous, wherein E = 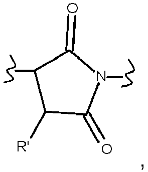 n is selected from the group of 1 and 2 and Q is selected from the group of phenyl, 1,4-phenylen, 1,3-phenylen, 4-Methyl-1,3-phenylen, 2,2,4-trimethyl-1,6-hexylen, 1,3-Benzenedimethylene (meta -CH₂-Ph-CH₂-), bis(1,4-phenylen)methane (para-CH₂-Ph-CH₂-) and 3,3'-Diethyl-5,5'-dimethyl-4,4'-diphenylenmethane.

The inventive functionalized cyclic carbonates will be advantageous, wherein E = -CH₂-CH₂-C(O)-, n is selected from the group of 1 and 2 and Q is selected from the group of -O-Y and -O-Z-O-.

The inventive functionalized cyclic carbonates will be advantageous, wherein E = -CH₂-CH₂-C(O)-, n is 1, R¹ is selected from H and methyl and Q is -OH.

The inventive functionalized cyclic carbonates will be advantageous, wherein E = -CH₂-CH₂-C(O)-, n is selected from the group of 1 and 2 and Q is selected from the group of -O-Y and -O-Z-O-, wherein Y is selected from the group of H, substituted or unsubstituted C₁-C₄₀ alkyl groups, substituted or unsubstituted saturated or unsaturated C₃-C₄₀ cycloalkyl groups, substituted or unsubstituted saturated alkylenheterocyclic groups containing 3 to 20 carbon atoms and one or more heteroatoms selected from the group of N, O, and S as ring members, substituted C₃-C₄₀ unsaturated alkylencycloalkyl groups and
Z which is selected from the group of substituted or unsubstituted C₁-C₄₀ alkylen groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded saturated substituted or unsubstituted C₅-C₄₀ alkylencycloalkylalkylen groups and divalently bonded substituted or unsubstituted C₁₇-C₃₀ alkylen-bisphenol A -alkylen groups.

The inventive functionalized cyclic carbonates will be advantageous, wherein E = -CH₂-CH₂-C(O)-, n =1 or 2 and Q is selected from the group of -NR²-Y, 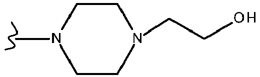 (4-(2-hydroxyethyl)piperazin-1-yl group, -NR²-Z-NR²-, -NR²-Z-O- and 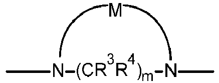 group wherein R², Y, Z, R³, R⁴, M and m are as defined above.

The inventive functionalized cyclic carbonates will be advantageous, wherein E = -CH₂-CH₂-C(O)-, n =1 or 2 and Q is selected from the group of -NR²-Y, 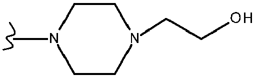 (4-(2-hydroxyethyl)piperazin-1-yl group, -NR²-Z-NR²-, -NR²-Z-O- and 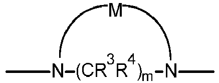 group, wherein
- R²: is selected from the group of H and substituted or unsubstituted C₁-C₄₀ alkyl groups,
- Y: is selected from the group of substituted or unsubstituted C₁-C₄₀ alkyl groups and substituted or unsubstituted C₆-C₄₀ aryl groups,
- Z: is selected from the group of substituted or unsubstituted C₁-C₄₀ alkylen groups, divalently bonded substituted or unsubstituted C₆-C₄₀ aryl groups and divalently bonded substituted or unsubstituted alkylenheterocyclicalkylen groups containing 4 to 20 carbon atoms and one or more heteroatoms selected from the group of N, O, and S as ring members,
- M: is -CH₂-CH₂-,
- R³ and R⁴: are H and
- m: is 2.

The inventive functionalized cyclic carbonates will be advantageous, wherein E = -CH₂-CH₂-C(O)-, n is 3 and Q is selected from the group of unsubstituted 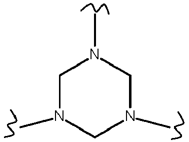 (1,3,5-triazinan) group, 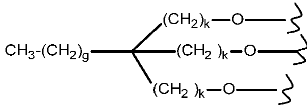 group, 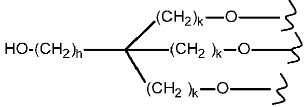 group and 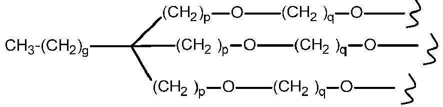 group wherein
- g: is a natural number selected from 1 to 33,
- k: is a 1,
- h: is a natural number selected from 1 to 15,
- p: is a 1 and
- q: is a natural number selected from 1 to 5.

The inventive functionalized cyclic carbonates will be advantageous, wherein E = -CH₂-CH₂-C(O)-, n ≥ 4 and Q is selected from the group of 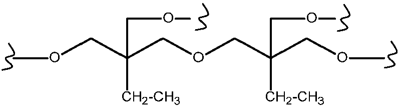 and

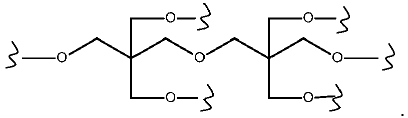

A further embodiment of the invention is a process for the preparation of the inventive functionalized cyclic carbonates of formula (I) 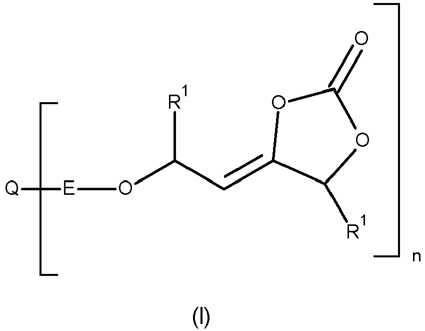 comprising the steps of:
a) reacting an alkynyl ether compound of formula (II) 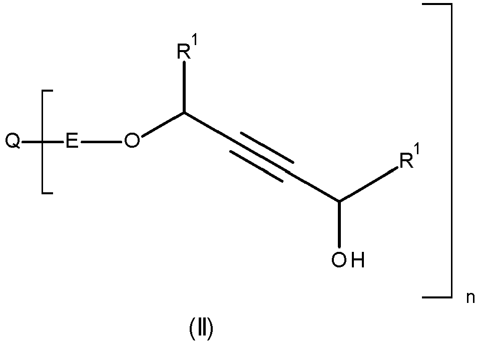 wherein n, R¹, E and Q are as defined above,
   with carbon dioxide in the presence of at least one transition metal catalyst TMC1, which comprises Ag as transition metal and at least one bulky ligand selected from the group of compounds of formula (III) and compound of formula (IV) 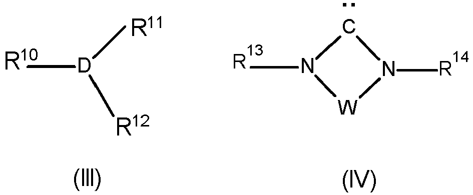 wherein
   - D: is P, As or Sb;
   - R¹⁰: is selected from the group of a substituted or unsubstituted C₃-C₄₀ cycloalkyl group, a substituted or unsubstituted C₂-C₄₀ heterocycloalkyl group, a substituted or unsubstituted C₆-C₄₀ aryl group, a substituted or unsubstituted C₂-C₄₀ heteroaromatic group, a substituted or unsubstituted C₃-C₄₀ cycloalkoxy group, a substituted or unsubstituted C₂-C₄₀ heterocycloalkoxy group, a substituted or unsubstituted C₆-C₄₀ aryloxy group and a C₂-C₄₀ heteroaryloxy group,
   - R¹¹ and R¹²: are independently of each other selected from the group of substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₂₀ heterocycloalkyl groups, substituted or unsubstituted C₆-C₄₀ aryl groups, substituted or unsubstituted C₂-C₄₀- heteroaromatic groups, substituted or unsubstituted C₆-C₁₀ fluoroaryl groups, substituted or unsubstituted C₂-C₂₀ alkynyl groups and substituted or unsubstituted C₇-C₄₀- alkylenaryl groups,
   - R¹³ and R¹⁴: are independently of each other selected from the group of substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₄₀ cycloalkoxy group, substituted or unsubstituted C₃-C₂₀ heterocycloalkyl groups, a substituted or unsubstituted C₂-C₄₀ heterocycloalkoxy group, substituted or unsubstituted C₆-C₄₀ aryl groups, substituted or unsubstituted C₆-C₄₀ aryloxy group, substituted or unsubstituted C₂-C₄₀- heteroaromatic groups, substituted or unsubstituted C₆-C₁₀ fluoroaryl groups, substituted or unsubstituted C₂-C₂₀ alkynyl groups and substituted or unsubstituted C₇-C₄₀- alkylenaryl groups, and
   - W: is a divalent bridging group selected from the group of -CR¹⁵=CR¹⁶-, -CR¹⁵=N-, -CR¹⁵R¹⁷-CR¹⁶R¹⁸- and -CR¹⁵R¹⁷-CR¹⁶R¹⁸-CR¹⁹R²⁰-,
   wherein
   R¹⁵, R¹⁶ R¹⁷, R¹⁸, R¹⁹ and R²⁰ are independently of each other selected from the group of hydrogen, substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₄₀ cycloalkoxy group, substituted or unsubstituted C₃-C₂₀ heterocycloalkyl groups, substituted or unsubstituted C₂-C₄₀ heterocycloalkoxy group, substituted or unsubstituted C₆-C₄₀ aryl groups, substituted or unsubstituted C₆-C₄₀ aryloxy group, substituted or unsubstituted C₂-C₄₀- heteroaromatic groups, substituted or unsubstituted C₆-C₁₀ fluoroaryl groups, substituted or unsubstituted C₂-C₂₀ alkynyl groups, substituted or unsubstituted C₇-C₄₀- alkylenaryl groups and two adjacent groups R¹⁵ and R¹⁶ and/or R¹⁸ and R¹⁹ together with the rest of the group W build up a monocyclic or polycyclic, substituted or unsubstituted, aliphatic or aromatic carbon or heteroatom comprising ring system containing 4 to 40 carbon atoms and wherein the heteroatom comprising ring system comprises one or more heteroatoms selected from the group of Si, Ge, N, P, O and S as ring members
b) separating the functionalized cyclic carbonates of formula (I) via distillation, extraction, precipitation and/or crystallization
or
for compounds of formula (I) with n = 1, E = -CH₂-CH₂-C(O)- and Q = -OH and compounds of formula (I) with n = 2, E= -CH₂-CH₂-C(O)- and Q= -NH-C₂-C₈-alkyl-NH-, the compounds of formula (I) with n=1, E= -CH₂-CH₂-C(O)- and Q = tert-butyl which are received after step a) and b) wherein an alkynylether compound of formula (II) with n =1, E = -CH₂-CH₂-C(O)- and Q = tert-butyl is used in step a) the resulting compound of formula (I) with n =1, E = -CH₂-CH₂-C(O)- and Q = tert-butyl after step b) will be hydrolysis with an acid selected from the group of anorganic acids and heterogene acid compounds to receive the compound of formula (I) with n = 1, E = -CH₂-CH₂-C(O)- and Q = -OH which will be purified by extraction and crystallization and the resulting product will react with an diisocyanate containing 2 to 13 C- atoms to receive the compound of formula (I) with n = 2, E=-CH₂-CH₂-C(O)- and Q= -NH-C₂-C₈-alkyl-NH-.

The inventive process will be advantageous, when R¹⁰ of formula (III) and R¹³ of formula (IV) is selected from the group of substituted or unsubstituted C₆-C₄₀ aryl group and a C₂-C₄₀ substituted or unsubstituted heteroaromatic group and R¹⁵, R¹⁶ R¹⁷, R¹⁸, R¹⁹ and R²⁰ in formula (IV) are H.

The inventive process will be advantageous, when R¹⁰ is substituted in at least one of the two ortho position of the ring cyclus relative to D with a group R^{10a},
- wherein R^{10a}: is selected form the group of substituted or unsubstituted C₆-C₃₄ aryl group, substituted or unsubstituted C₁-C₁₀ alkoxy group, substituted or unsubstituted C₂-C₁₂ dialkylamino group and where R^{10a} forms with an adjacent group substituting R¹⁰ in the meta position of the ring cyclus together with the atoms connecting them a monocyclic or polycyclic, substituted or unsubstituted aliphatic, aromatic or heteroatom comprising ring system comprising 4 to 40 carbon atoms wherein the heteroatoms are selected from the group of Si, Ge, N, P, O and S.

The inventive process will be advantageous, wherein the transition metal catalyst TMC1 is prepared in situ by using a transition metal compound comprising Ag, which does not comprise any bulky ligand, the compound of formula (III) or (IV) as bulky ligand or the protonated form of the compound of formula IV represented by formula (V) 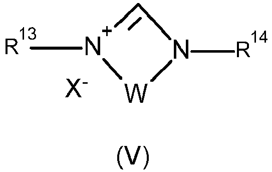 together with a base, wherein R¹³, R¹⁴ and W are defined as above and X- is an anion selected from the group of F-, CI-, Br-, I-, PF₆-, NTf₂- ([(CF₃SO₂)₂N]⁻), OTf- (F₃CS(=O)₂-O⁻), MeSO₂O⁻, Me-SO₃O⁻, OAc-, CO₂⁻ and [AlCl₄]⁻.

The inventive process will be advantageous, wherein the transition metal compound is selected from the group of AgOAc, AgF, Ag₂O and Ag₂CO₃.

The inventive process will be advantageous, wherein the bulky ligand is a compound of formula (III).

The inventive process will be advantageous, wherein the bulky ligand is a compound selected from the group of a compound A' to P and mixtures thereof

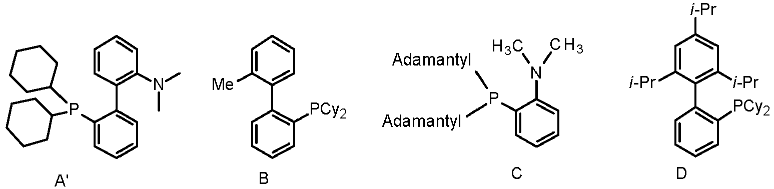

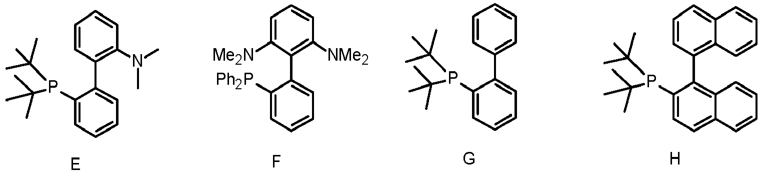

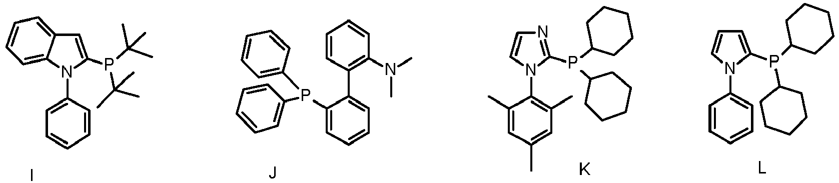

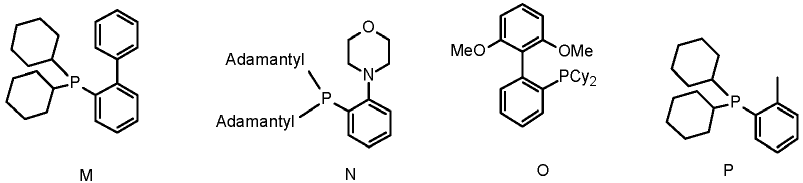

The inventive process will be advantageous, wherein the molar ratio of the bulky ligand to Ag of transition metal catalyst TMC1 is in the range of 0.4 to 1.2.

The inventive process will be advantageous, wherein the amount of transition metal catalyst TMC1 used in step a) based on the amount of alkynyl ether compound of formula (II) is in the range from 0.005 to 5 mol%.

The inventive process will be advantageous, wherein the pressure during step a) is in the range from 1 to 50 bar.

The inventive process will be advantageous, wherein the pressure during step a) is in the range from 0°C to 100°C.

The inventive process will be advantageous, wherein the transition metal catalyst TMC1 is recycled to step a) after step b).

A further embodiment of the invention is the use of the cyclic carbonates of formula (I) as precursors of monomers in polymer reactions or as active monomers in polymerization reaction if the compound of formula (I) carries a further hydroxy group.

The term "alkyl" group as used in the present application comprises linear or singly or multiply branched saturated hydrocarbons. Preferably are linear, singly or multiply branched saturated C₁-C₂₂-alkyl groups, more preferably are linear, singly or multiply branched saturated C₁-C₁₀ alkyl, much more preferably are linear, singly or multiply branched C₁-C₈ alkyl groups selected from the group of methyl, ethyl, n-propyl, isopropyl, n-butyl, sec-butyl, tert.-butyl, 1,1-dimethylethyl n-pentyl, 2-methylbutyl, 2,2-dimethylpropyl, n-hexyl, 2-methylpentyl, 3-methylpentyl, 2,2,-dimethylbutyl, 2,3-dimethylbutyl, n-heptan, 2-methylhexyl, 3-methylhexyl, 2,2-dimethylpentyl, 2,3-dimethylpentyl, 3,3-dimethylpentyl, 2,4-dimethylpentyl, 3-ethylpentyl, 2,2,3 trimethylbutyl, n-octyl, 2-methylheptyl, 3-methylheptyl, 4-methylheptyl, 2,2-dimethylhexyl, 2,3-dimethylhexyl, 2,4-dimethylhexyl, 2,5-dimethylhexyl, 3,3,-dimethylhexyl, 3,4-dimethylhexyl, 3-ethylhexyl, 2,2,3-trimethylpentyl, 2,2,4-trimethylpentyl, 2,3,3-trimethylpentyl, 2,3,4-trimethylpentyl, 3-ethyl-2-methylpentyl, 3-ethyl-3-methylbutyl, 2,2,3,3-tetramethylbutyl, most preferred are methyl, ethyl, n-propyl, isopropyl, n-butyl, sec-butyl, tert.-butyl, n-pentyl, 2-methylbutyl, 2,2-dimethyl propyl, n-hexyl, 2-methylpentyl, 3-methylpentyl, 2,2,-dimethylbutyl, 2,3-dimethylbutyl. Most preferred C₁-C₅ alkyl groups are selected from the group of methyl, ethyl, n-propyl, isopropyl, n-butyl, sec-butyl, tert.-butyl, 1,1-dimethylethyl, n-pentyl, 2-methylbutyl, 2,2-dimethylpropyl. Much more preferably are C₁-C₅ alkyl groups selected from the group of methyl, ethyl, n-propyl, isopropyl, n-butyl, sec-butyl, tert-butyl, 1,1-dimethylethyl and 2,2-dimethylpropyl.

The term "terminal alkoxy" group as used in the present application comprises the alkyl group that is bonded via an O atom to the rest of the molecule in question and which is exclusively used as group in the bulky ligands of the transition metal catalyst TMC1 compounds of formula (III), (IV) and/or (V). Preferably are linear, singly or multiply branched saturated C₁-C₁₂ terminal alkoxy groups, more preferably are linear, singly or multiply branched saturated C₁-₁₀ terminal alkoxy groups, much more preferably are linear, singly or multiply branched saturated C₁-C₈ terminal alkoxy groups selected from the group of methoxy, ethoxy, n-propoxy, isopropoxy, n-butoxy, sec-butoxy, tert.-butoxy, n-pentoxy, 2-methylbutoxy, 2,2,-dimethylpropoxy, n-hexoxy, 2-methylpentoxy, 3-methylpentoxy, 2,2,-dimethylbutoxy, 2,3-dimethylbutoxy, n-heptoxy, 2-methylhexoxy, 3-methylhexoxy, 2,2-dimethylpentoxy, 2,3-dimethylpentoxy, 3,3-dimethylpentoxy, 2,4-dimethylpentoxy, 3-ethylpentoxy, 2,2,3 trimethylbutoxy, n-octoxy, 2-methylheptoxy, 3-methylheptoxy, 4-methylheptoxy, 2,2-dimethylhexoxy, 2,3-dimethylhexoxy, 2,4-dimethylhexoxy, 2,5-dimethylhexoxy, 3,3,-dimethylhexoxy, 3,4-dimethylhexoxy, 3-ethylhexoxy, 2,2,3-trimethylpentoxy, 2,2,4-trimethylpentoxy, 2,3,3-trimethylpentoxy, 2,3,4-trimethylpentoxy, 3 ethyl-2-methylpentoxy, 3-ethyl-3-methylbutoxy, 2,2,3,3-tetramethylbutoxy, most preferred are methoxy, ethoxy, n-propoxy, isopropoxy, n-butox, sec-butoxy, tert.-butoxy, n-pentoxy, 2-methylbutoxy, 2,2-dimethyl propoxy, n-hexoxy, 2-methylpentoxy, 3-methylpentoxy, 2,2,-dimethylbutoxy, 2,3-dimethylbutoxy. Most preferred C₁-C₄ terminal alkyloxy groups are selected from the group of methoxy, ethoxy, n-propoxy, isopropoxy, n-butoxy, sec-butoxy, tert.-butoxy. Much more preferably are C₁-C₄ terminal alkyloxy groups selected from the group of methoxy ethoxy and tert-butoxy.

The term "alkylen" group as used in the present application does not mean alkenes having double bonds but comprises a C₁-C₂₀, preferably C₁- C₁₀, more preferably C₁-C₆ divalent bonded linear or with C₁-C₄ alkyl groups substituted saturated hydrocarbons having free valencies on adjacent or terminal carbon atoms. Preferably the alkylen group is selected from the group of methylene (-CH₂-), ethylene (-CH₂-CH₂-), n-propylene (-CH₂-CH₂-CH₂-), isopropylene (-CH(CH₃)-CH₂-), 2,2,-dimethylpropylene (-CH₂-C(CH₃)₂-CH₂-), n-butylene (-CH₂-CH₂-CH₂-CH₂-) n-pentylene (-CH₂-CH₂-CH₂-CH₂-CH₂) and n-hexylene (-CH₂-CH₂-CH₂-CH₂-CH₂-CH₂-).

The term "alkylhydroxide" group as used in the present application comprises C₁-C₂₂ alkyl groups where at least one H atom of the alkyl chain is substituted by one or more OH groups. Preferably the alkylhydroxide is selected from the group of methyl hydroxide, ethylhydroxide, n-propylhydroxide, 2-methylpropylhydroxid, 2,2-dimethylpropylhydroxide, 2-hydroxypropyl, 1-dodecanylhydroxid (lauryl), 1-hexandecanylhydroxid (cetyl), 1-octadecanylhydroxid (stearyl) and 1-docosanylhydroxid (behenyl). More preferably are methylhydroxide, ethylhydroxide, 2-methylpropylhydroxid, 2,2-dimethylpropylhydroxide, and 2-hydroxypropyl.

The term "cycloalkyl" group as used in the present application comprises C₃-C₄₀ containing monocyclic or polycyclic aliphatic hydrocarbon groups wherein the term "polycyclic" comprises two or more cycloalkyl rings which are independently to each other connected by normal C-C bond, by a spiro-bond (two cycloaliphatic rings share one sp³ hybridized C-atom), by sharing at least one C-C bond, which is part of two cycloaliphatic ring systems (condensed ring system) or by sharing at least two C-atoms in at least two cycloaliphatic ring systems where a C₁-C₃-alkylen group is in between (bridged ring systems). Preferably the monocyclic aliphatic groups are selected from the group of cyclopropanyl, cyclobutanyl, cyclopentanyl, cyclohexanyl, cycloheptanyl and cyclooctanyl. More preferred monocyclic aliphatic groups are selected from the group of cyclobutanyl, cyclopentenyl and cyclohexanyl. The preferred polycyclic aliphatic groups are selected from the group of bicyclopentanyl, bicyclohexanyl, spiropentanyl, spiroheptanyl, spiroundecanyl, spiro [4.5] decanyl, dispiro-[4.2.2.5.2]pentadecanyl, decalinyl, bicyclo[2.2.1]heptyl, bicyclo[3.1.1]heptyl, tricyclo[5.2.1.0]decanyl, Trimethylbicyclo[2.2.1]hept-2-yl., Tricyclo[5.2.1.0]decanyl. More preferred polycyclic aliphatic groups are selected from the group of bicyclopentanyl, bicyclo[2.2.1]heptyl and tricyclo[5.2.1.0]decanyl.

The term "cycloalkoxy" group as used in the present application comprises the cycloalkyl group that is bonded via an O atom to the rest of the molecule in question. Preferably the monocyclic aliphatic alkoxy groups comprising 3 to 40 carbon atoms and are selected from the group of cyclopropanoxy, cyclobutanoxy, cyclopentanoxy, cyclohexanoxy, cycloheptanoxy and cyclooctanoxy. The preferred polycyclic aliphatic alkyloxy groups comprising 4 to 40 carbon atoms and are selected from the group of bicyclobutanoxy, bicyclopentanoxy, bicyclohexanoxy, spiropen-tanoxy, spiroheptanoxy, spiroundecanoxy, spiro [4.5] decanoxy, dispiro-[4.2.2.5.2]pentadecanoxy, decalinoxy, norbornanoxy.

The term "alkylencycloalkyl" group used in the present application comprises a C₁ - C₁₅ containing alkylen group wherein at least one H is substituted by a mono- or polycyclic C₃ - C₃₉ containing cycloalkyl group wherein the sum of the carbon atoms in the alkylencycloalkyl group is not higher than 40. Preferably are the alkylencycloalky groups wherein the alkylen group is selected from the group of methylen, ethylen, n-propylen, isopropylen, n-butylen, sec-butylen and tert.-butylen group and the cycloalkyl group is selected from C₃- C₁₂ cycloalkyl groups. More preferably the alkylencylcoalkyl group is selected from the group of methylencyclopropan, methylencyclobutan, methylencyclopentan, methylencyclohexan, methylenbicyclopentan, methylenbicyclohexan, methylennorbornan, ethylencyclopentan, ethylencyclohexan, ethylenbicyclopentan, ethylenbicyclohexan, ethylennorbornan, n-propylencyclopropan, isopropylencyclohexan, isopropylencyclopentan, isopropylenbicyclohexan, isopropylennorbornan, n-butylencyclopentan, n-butylencyclohexan, n-butylenbicyclopentan, n-butylenbicyclohexan, n-butylennorbornan, secbutylenbicyclopentan, sec-butylenbicyclohexan, sec-butylenspiropentan, sec-butylennorbornan, tert-butylencyclopentan, tert-butylencyclohexan, tert-butylenbicyclopentan, tertbutylenbicyclohexan, tert-butylennorbornan. Most preferably the alkylencycloalkyl group is selected from the group of methylencyclopentan, methylencyclohexan, methylenbicyclopentan and isopropylencyclohexan.

The term "alkylencycloalkylalkylen" group used in the present application comprises a C₅-C₄₀ containing group which is divalent and comprises a cycloalkyl group in the center and two identical or different alkylen groups at each side. The alkylencycloalkylalkylen group is a bridging group wherein the alkylen groups at each side are bonded either to two different or identical groups or which are bonded to the same or to different atoms of one group. The alkylen groups are independently selected from the group of methylen, ethylen, propylene and isopropylen. The cycloalkyl group is selected from the group of 1,2-cyclopropan, 1,2-, 1,3-, 1,4-cyclobutan, 1,2-, 1,3-clyopentan, 1,2-, 1,3-, 1,4-cyclohexan. The numbers in front of the cycloalkyl groups symbolize the C-atoms of the cycloalkylring which are bonded to the alkylen groups. Preferably alkylencycloalkylalkylen groups are selected from the group of 1,3-dimethylencyclopentan, 1,3 dimethylencyclohexan, 1,2-dimethylencyclohexan, 1,4-dimethylencyclohexan, 1,3-diethylencyclopentan, 1,4-diethylencyclohexan, 1,3-dipropylencyclopentan, 1,4-dipropylencyclohexan, 4,8-bis(methylene)tricyclo[5.2.1.0^{2,6}]decan. More preferably the alkylencycloalkylen groups are selected from the group of 1,3 dimethylencyclohexan, 1,2-dimethylencyclohexan and 1,4-dimethylencyclohexan.

The term "cycloalkylalkylencycloalkyl" group used in the present application comprising a C₇-C₄₀ atoms containing group which is divalent and comprises an alkylen chain in the center, wherein the alkylen chain is selected from the group of methylene, ethylene, propylene, isopropylene and at least two mono-, two poly- or one mono- and one polycycloalkylring are bonded at each side of the alkylen chain. The cycloalkyl groups at each side of the alkylen chain are the same or different. The mono- and/or polycyclic rings at each side of the alkylen chain will be the connection to a further group at each side of the mono- and/or polycyclic group which is either identical or different. Preferably monocycloalkylrings are independently from each other selected from the group of cyclopropanyl, 1,2- and 1,3-cyclobutanyl, 1,2- and 1,3-cyclopentanyl and 1,2-, 1,3- and 1,4-cylohexanyl. More preferably monocycloalkyrings are independently from each other selected from the group of 1,3-cyclobutanyl, 1,3-cyclopentanyl, 1,2- and 1,4-cylohexanyl. The numbers in front of the cycloalkyl groups symbolize the C-atoms of the cycloalkylring which are bonded on the one side to the alkylen group and on the other side to the further group. Preferably polycycloalkylrings are independently from each other selected from the group of tricyclo[5.2.1.0^{2,6}]decanyl, 2-norbornyl, bornyl, isobornyl, 2-decahydronaphthyl, tetracyclo-[4.4.0.1^{2,5}.1^{7,10}]docecyl. Preferably cycloalkylalkylencycloalkyl groups are selected from the group of dicyclopropanylmethan, 1,2- and 1,3-dicyclobutanylmethan, 1,2- and 1,3-dicyclopentanylmethan, 1,2-, 1,3- and 1,4-dicyclohexanylmethan, dicyclopropanylethan, 1,2-and 1,3-dicyclobutanylethan, 1,2- and 1,3-dicyclopentanylethan, 1,2-, 1,3- and 1,4-dicyclohexanylethan, dicyclopropanylpropan, 1,2- and 1,3-dicyclobutanylpropan, 1,2- and 1,3-dicyclopentanylpropan, 1,2-, 1,3- and 1,4-dicyclohexanylpropan, 1,2- and 1,3-dicyclobutanylisopropan, 1,2- and 1,3-dicyclopentanylisopropan, 1,2-, 1,3- and 1,4-dicyclohexanylisopropan, cyclopropanyl-1,2- or 1,3-cyclobutanylmethan, cyclopropanyl-1,2- or 1,3-cyclopentanylmethan, cyclopropanyl-1,2- or 1,3- or 1,4-cyclohexanylmethan, 1,2- or 1,3-cyclobutanyl-1,2- or 1,3-cyclopentaylmethan, 1,2- or 1,3-cyclobutanyl-1,2 or 1,3 or 1,4-cyclohexanylmethan, 1,2- or 1,3-cyclopentanyl1,2 or, 1,3 or 1,4-cyclohexanylmethan, cyclopropanyl-1,2 or 1,3-cyclobutanylethan, cyclopropanyl-1,2 or, 1,3-cyclopentanylethan, cyclopropanyl1,2 or 1,3 or 1,4-cyclohexanylethan, 1,2 or 1,3-cyclobutanyl-1,2 or 1,3-cyclopentaylethan, 1,2 or 1,3-cyclobutanyl-1,2 or 1,3 or 1,4-cyclohexanylethan, 1,2- or 1,3-cyclopentanyl-1,2 or 1,3- or 1,4-cyclohexanylethan, cyclopropanyl-1,2- or 1,3-cyclobutanylpropan, cyclopropanyl-1,2- or 1,3-cyclopentanylpropan, cyclopropanyl-1,2- or 1,3- or 1,4-cyclohexanylpropan, 1,2- or 1,3-cyclobutanyl-1,2 or 1,2-cyclopentaylpropan, 1,2- or 1,3-cyclobutanyl-1,2 or 1,3 or 1,4-cyclohexanylpropan, 1,2 or 1,3-cyclopentanyl-1,2 or 1,3 or 1,4-cyclohexanylpropan. More preferably cycloalkylalkylencycloalkyl groups are selected from the group of 1,4-dicyclohexanylmethan, 1,3-dicyclopentanylethan and 1,4-dicyclohexanylisopropan.

The term "unsaturated" cycloalkyl, alkylencycloalkyl, alkylencycloalkylalkylen, cycloalkylalkylencycloalkyl or alkylenheterocyclicalkylen group used in the present application comprises C₃-C₄₀ cycloalkyl, a C₄-C₄₀ alkylencycloalkyl, a C₅-C₄₀ alkylencycloalkylalkylen, a C₇-C₄₀ cycloalkylalkylencycloalkyl or a C₄-C₄₀ alkylenheterocyclicalkylen groups that include at least one C-C double bond inside of one or more of the rings, wherein no double bond is located at the bridge head of a bridged bi- or multicycloalkyl ring system. Preferably unsaturated monocyclic cycloalkyl groups are selected from the group of cyclopentenyl, cyclohexenyl, cyclooctenyl and cyclododecenyl. Preferably unsaturated polycyclic cycloalkyl group are selected from the group of dihydrodicyclopentadienyl and norbornenyl. Preferably unsaturated monocyclic alkylencycloalkyl groups are selected from the group of (cyclohex-3-en-1-yl)methylene and (4-methyl-1-cyclohexene)isopropylene. Preferably unsaturated polycyclic alkylencycloalkyl groups are selected from the group of Bicyclo [2.2.1]hept-5-ene-2-methylene, 2-Methyl-bicyclo [2.2.1]hept-5-ene-2-methylene, 3a,4,5,6,7,7a-Hexahydro-4,7-methano-1*H*-indene-5-methylene and 3a,4,5,6,7,7a-Hexahydro-4,7-methano-1*H*-indene-6-methylene. Preferably unsaturated monocyclic alkylencyloalkylalkylen groups are selected from the group of 4-Cyclopentene-1,3-dimethylene and 2-Cyclohexene-1,4-dimethylene. Preferably unsaturated monocyclic alkylenheterocyclicalkylen groups is 2,5 furandimethylene.

The term "aryl" group used in the present application comprises C₆-C₄₀ containing mono- or polycyclic aromatic hydrocarbon groups wherein the term "polycyclic aromatic" comprises two or more rings wherein the whole ring system is aromatic and in which the rings are connected by a normal C-C-bond or by sharing at least one C-C bond, which is part of two rings (condensed ring systems). Preferably mono- or polycyclic aromatic hydrocarbon groups are selected from the group of phenyl, biphenyl, naphthyl, anthracenyl, phenanthrenyl, indenyl, as-indacenyl, fluorenyl. Most preferred are phenyl and naphthyl.

The term "aryloxy" group as used in the present application comprises a C₆ to C₄₀ aryl group that is bonded via an O atom to the rest of the molecule in question. Preferably mono- or polycyclic aromatic hydrocarbonoxy groups are selected from the group of phenyloxy, biphenyloxy, naphthalenoxy, anthracenoxy, phenanthrenoxy, indenoxy, as-indacenoxy, fluorenoxy)

The term "alkylenaryl" group used in the present application comprises a C₇ to C₄₀ containing group that is build up by an alkylen chain that is bonded to a mono- or polycyclic aryl group. Preferably are the alkylenaryl groups wherein the alkylen chain is selected from the group of methylene, ethylene, n-propylene, iso-propylene, n- butylene, sec-butylene and tert.-butylene group and the aryl group is selected from C₆ to C₁₂ aryl groups. More preferably the alkylenaryl group is selected from the group of benzyl, methylenbiphenyl, methylennaphthalenyl, methylenanthracenyl, methylenphenanthrenyl, methylenindenyl,methylene-as-indacenyl, methylenfluorenyl, ethylenphenyl, ethylenbicyclophenyl, ethylennaphthalenyl, ethylenanthracenyl, ethylenphenanthrenyl, ethylenindenyl, ethylene-as-indacenyl, ethylenfluorenyl, n-propylenphenyl, n-propylenbiphenyl, n-propylenanthracenyl, n-propylenphenanthrenyl, n-propylenindenyl, n-propylene-as-indacenyl, n-propylenfluorenyl, iso-propylenphenyl, iso-propylenbiphenyl, isopropylenantracenyl, isopropylenphenantrenyl, iso-propylenindenyl, iso-propylene-as-indacenyl, iso-propylenfluorenyl, n-butylenphenyl, n-butylenbicyclophenyl, n-butylennaphthalenyl, n-butylenanthracenyl, n-butylenphenanthrenyl, n-butylenindenyl, n-butylene-as-indacenyl, n-butylenfluorenyl, sec-butylenphenyl, sec-butylenbicyclophenyl, sec-butylennaphthalenyl, secbutylenanthracenyl, sec-butylenphenanthrenyl, sec-butylenindenyl, sec-butylene-as-indacenyl, sec-butylenfluorenyl, tert.-butylenphenyl, tert.-butylenbicyclophenyl, tert.-butylennaphthalenyl, tert.-butylenanthracenyl, tert.-butylenphenanthrenyl, tert.-butylenindenyl, tert.-butylene-as-indacenyl, tert.-butylenfluorenyl. Most preferred alkylenaryl groups are selected from benzyl, ethylenphenyl, iso-propylenphenyl and tert.-butylenphenyl.

The term "arylalkylenaryl" group used in the present application comprising a C₁₃-C₄₀ atoms containing group which is divalent and comprises at least two aryl groups which are connected via an C₁-C₁₀ containing alkylen chain. The connection to the arylalkylenaryl group will be built to each of the two aryl groups. Preferably the alkylen chain, which is in the center of the two aryl groups, is selected from the group of methylene, ethylene, propylene and isopropylene. At each side of the center alkylen chain an aryl group is connected. These aryl groups can be identical or different. As each aryl group is further connect to a further group, which also can be the same or different, at each side of the arylalkylenaryl group, each aryl group has two connection carbon atoms which are identified here by the number of the carbon atom of the aryl group that build up the connection to the center alkylen group as well as the number of the carbon atom that build up the connection to the further group on the other side of the aryl group. Preferably the aryl groups are independently selected from the group of 1,2-, 1,3- and 1,4-phenyl, 1,2-, 1,3-, 1,4-, 1,5-,1,6-, 1,7-, 1,8-, 2,3-, 2,4-, 2,5-, 2,7- 2,8-, 3,4-, 3,5-, 3,6-, 3,7- and 3,8-naphthyl, 1,2-, 1,3-, 1,4-, 1,5-, 1,6-, 1,7-, 1,8-, 1,9-, 1,10-, 2,3- 2,4-, 2,5-, 2,6-, 2,7-, 2,8-, 2,9-, 2,10-, 3,4-, 3,5-, 3,6-, 2,7- 3,8-, 3,9- and 3,10-anthracenyl. Preferably arylalkylenaryl groups are selected from the group of bis(1,4-phenyl)methane, bis(2,5-naphthyl)methan, bis(2,7-anthracenyl)methan, 1,1-bis(1,4-phenyl)ethane, 1,1-bis(2,5-naphthyl)ethan, 1,1-bis(2,7-anthracenyl)ethan, 1,2-bis(1,4-phenyl)ethane, 1,2-bis(2,5-naphthyl)ethan, 1,2-bis(2,7-anthracenyl)ethan, 1,3-bis(1,4-phenyl)propan, 1,3-bis(2,5-naphthyl)propan, 1,3-bis(2,7-anthracenyl)propan, 1,2-bis(1,4-phenyl)propan, 1,2-bis(2,5-naphthyl)propan, 1,2-bis(2,7-anthracenyl)propan, 1,1-bis(1,4-phenyl)propan, 1,1-bis(2,5-naphthyl)propan, 1,1-bis(2,7-anthracenyl)propan bis(1,4-phenyl)isopropan, bis(2,5-naphthyl)isopropan, bis(2,7-anthracenyl)isopropan, (1,4-phenyl),(2,5-naphthyl)methane, (1,4-phenyl),(2,7-anthracenyl)methan, (2,5-napthyl),(2,7-antracenyl)methan, 1-(1,4phenyl),2-(2,5-naphthyl)ethane, 1-(1,4-phenyl),2-(2,7anthracenyl)ethan, 1-(2,5-napthyl),2-(2,7-antracenyl)ethan, 1-(1,4phenyl),1-(2,5-naphthyl)ethane, 1-(1,4-phenyl),1-(2,7-anthracenyl)ethan, 1-(2,5-napthyl),1-(2,7-antracenyl)ethan, 1-(1,4-phenyl),3-(2,5-naphthyl)propan, 1-(1,4-phenyl),3-(2,7-anthracenyl)propan, 1-(2,5-napthyl),3-(2,7-antracenyl)propan, 1-(1,4-phenyl),2-(2,5-naphthyl)propan, 1-(1,4-phenyl),2-(2,7-anthracenyl)propan, 1-(2,5-napthyl),2-(2,7-antracenyl)propan, 1-(1,4-phenyl),1-(2,5-naphthyl)propan, 1-(1,4-phenyl),1-(2,7-anthracenyl)propan, 1-(2,5-napthyl),1-(2,7-antracenyl)propan, (1,4-phenyl)-(2,5-naphthyl)isopropan, (1,4-phenyl)-(2,7-anthracenyl)isopropan, (2,5-napthyl)-(2,7-antracenyl)isopropan, Most preferred arylalkyenaryl groups are selected from the group of bis(1,4-phenyl)isopropan.

The term "alkylenarylalkylen group" used in the present application comprising C₈-C₄₀ atoms containing group which is divalent and comprises at least two alkylen groups which are connected via one aryl group. The aryl group is in the center of the alkylenarylalkylen group and has two different carbon atoms in the ring to which each alkylen group is bonded. The two carbon atoms of the center aryl group are identified by the numbers of the two carbon atoms in front of the aryl group to which the same or different alkylen groups are bonded. The alkylen groups are independently of each other selected from the group of methylene, ethylene, propylene, and iso-propylene. The center aryl group is selected from the group of 1,2-phenyl, 1,3-phenyl, 1,4-phenyl. Preferred alkylenarylalkylen group is selected from the group of 1,2- dimethylenphenyl (-CH₂-1,2-phenyl-CH₂-) and 1,4-dimethylenphenyl (-CH₂-1,4-phenyl-CH₂-) group.

The term "heterocyclic group" used in the present application comprises saturated or unsaturated heterocyclic groups that contains 2 to 20 carbon atoms and one or more heteroatoms selected from the group of Si, Ge, P, N, O and S as ring members. These heterocyclic groups can be divided in heterocycloalkyl groups and unsaturated heterocyclic groups. The term "unsaturated heterocyclic group" used in the present application comprises partially unsaturated heterocyclic groups as well as heteroaromatic groups. All of these heterocyclic groups can be a monoheterocyclic as well as a polyheterocyclic groups wherein at least in one cyclus at least one heteroatom is included. The heterocyclic groups used in inventive functionalized cyclic carbonates of formula (I) as well as in the alkynyl ether compounds of formula (II) only comprise heteroatoms selected from the group of N, O and S. Preferred heterocycloalkyl groups for the inventive functionalized cyclic carbonates of formula (I) as well as in the alkynyl ether compounds of formula (II) are selected from the group of ethylenoxidyl (oxiranyl), ethylensulfidyl (thiiranyl), ethyleniminyl (aziridinyl), trimethylenoxidyle (oxetanyl), trimethylensulfidyl (thietanyl), 1,3-propyleniminyl (azetidinyl), tetrahydrofuranyl (oxolanyl), tetrahydrothiophenyl (thiolanyl), pyrrolidyl (azolidinyl), tetrahydropyranyl (oxanyl), tetrahydrothiopyranyl (thianyl), piperidinyl (azinanyl), hexamethylenoxidyl (oxepanyl), hexamethylensulfidyl (thiepanyl), tetrahydro-1,4,-oxazinyl (morpholin), tetrahydro-1,3,-oxazinyl, tetrahydro-1,2,-oxazinyl hexamethyleniminyl (azepanyl), 1,3-dioxolanyl, 1,3-dithiolanyl, 1,3-oxathiolanyl, 1,3-oxaazolidnyl, 1,3-azathiolanyl, 1,3-dioxanyl, 1,4-dioxanyl, 1,3,5-trioxanyl, 1,3-dithianyl, 1,4-dithianyl, 1,3,5-trithianyl, 1,3-diazinanyl, 1,4-diazinanyl, 1,2,3-trianzinanyl, 1,2,4-triazinanyl, 1,3,5-triazinanyl (hexahydro-1,3,5-triazinyl), 1,2-oxazinanyl, 1,3-oxazinanyl, 1,4-oxazinanyl, octahydro-1-benzofuranyl, octahydro-2-benzofuranyl, hexahydro-furo[3,2-b]furanyl. More preferred heterocycloalkyl groups are selected from the group of ethylenoxidyl (oxiranyl), trimethylenoxidyle (oxetanyl), tetrahydrofuranyl (oxolanyl), piperidinyl (azinanyl), 1,3-dioxolanyl, 1,3-oxaazolidnyl, 1,3-dioxanyl, 1,3,5-trioxanyl, 1,3,5-triazinanyl (hexyhydro-1,3,5-triazinyl) and hexahydro-furo[3,2-b]furanyl. More preferred heterocycloalkyl groups are selected from the group of ethylenoxidyl (oxiranyl), trimethylenoxidyle (oxetanyl), tetrahydrofuranyl (oxolanyl), piperidinyl (azinanyl), 1,3-dioxolanyl, 1,3-oxaazolidnyl, 1,3-dioxanyl, 1,3,5-trioxanyl, 1,3,5-triazinanyl (hexyhydro-1,3,5-triazinyl) and hexahydro-furo[3,2-b]furanyl. Preferred heteroaromatic or heteroaryl groups, which means the same, for the inventive functionalized cyclic carbonates of formula (I) as well as in the alkynyl ether compounds of formula (II) are selected from the group of pyrrolyl (2-pyrrolyl), pyrazolyl (3-pyrazolyl), pyrrolium, imidazolyl (1-imi_dazolyl, 2-imidazolyl, 4-imidazolyl, 5-imidazolyl), oxazolium, isothiazolyl, triazolyl (1,2,3-triazol-1-yl, 1,2,3-triazol-2-yl 1,2,3-triazol-4-yl, 1,2,4- triazol-3-yl), diazolyl (1-oxa-2,3-diazolyl, 1-oxa-2,4-diazolyl groups, 1-oxa-2,5-diazolyl, 1-oxa-3,4-diazolyl, 1-thia-2,3-diazolyl, 1-thia-2,4-diazolyl, 1-thia-2,5-diazolyl, 1-thia-3,4-diazolyl), tetrazolyl, pyridinyl, oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyradizinyl, pyridiniumyl, pyrimidinium, pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), indazolyl, quinolyl (2- quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4- isoquinolyl, 5-isoquinolyl, 6-isoquinolyl, 7-isoquinolyl, 8-isoquinolyl), benzo[b]furanyl (2-benzo[b]furanyl, 3-benzo[b]furanyl, 4-benzo[b]furanyl, 5-benzo[b]furanyl, 6-benzo[b]furanyl, 7-benzo[b]furanyl), 2,3-dihydro-benzo[b]furanyl (2-(2,3-di_hydro-benzo[b]furanyl), 3-(2,3-dihydro-benzo[b]furanyl), 4-(2,3-dihydro-benzo[b]furanyl), 5-(2,3-dihydrobenzo-[b]fura_nyl), 6-(2,3-dihydro-benzo-[b]furanyl), 7-(2,3-dihydro-benzo[b]furanyl)), benzo[b]thiophenyl (2-benzo[b]thiophenyl, 3- benzo[b]thiophenyl, 4-benzo[b]thiophenyl, 5-benzo[b]thiophenyl, 6-benzo[b]thiophenyl, 7-benzo[b]thiophenyl), 2,3-di_hydro-benzo[b]thiophenyl (2-(2,3-dihydro-benzo[b]thiophenyl), 3-(2,3-dihydro-benzo[b]thiophenyl), 4-(2,3-dihydroben_zo[b]thiophenyl), 5-(2,3-dihydrobenzo[b]thiophenyl), 6-(2,3-dihydro-benzo[b]thiophenyl), 7-(2,3-dihydro-benzo[b]thi_ophenyl)), 4,5,6,7-tetrahydro-benzo[b]thiophenyl (2-(4,5,6,7-tetrahydro-benzo[b]thiophenyl), 3-(4,5,6,7-tetrahydro-ben_zo[b]thiophenyl), 4-(4,5,6,7-tetrahydro-benzo[b]thiophenyl), 5-(4,5,6,7-tetrahydro-benzo[b]thiophenyl), 6-(4,5,6,7-tetrahydro-benzo[b]thiophenyl), 7-(4,5,6,7-tetrahydrobenzo[b]thiophenyl)), thieno[2,3-b]thiophenyl, 4,5,6,7-tetrahydrothieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tetrahydrothieno[2,3-c]pyridyl), 7-(4,5,6,7-tetrahydro-thieno[2,3-c]pyridyl)), indolyl (1-indolyl, 2-indolyl, 3-indolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7- isoindolyl), 1,3-dihydro-isoindolyl (1-(1,3-dihydro-isoindolyl), 2-(1,3-dihydroisoindolyl), 3-(1,3-dihydro-isoindolyl), 4-(1,3- dihydro-isoindolyl), 5-(1,3-dihydro-isoindolyl), 6-(1,3-dihydro-isoindolyl), 7-(1,3-dihydro-isoindolyl)), indazole (1-indazolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxazolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), benzo-[1,2,5]oxadiazolyl, (4-benzo[1,2,5]oxadiazole, 5-benzo[1,2,5]oxadiazole), carbazolyl (1-carbazolyl, 2-carbazolyl, 3-carbazolyl, 4-carbazolyl), piperidinyl (2-piperidinyl, 3-piperidinyl, 4-piperidinyl), pyrrolidinyl (1-pyrrolidinyl, 2-pyrrolidinyl, 3- pyrrolidinyl), benzotriazolyl, pyrrolo[2,3-b]pyridinyl, pyrrolo[2,3-c]pyridinyl, pyrrolo[3,2-c]pyridinyl, pyrrolo[3,2-b]pyridinyl, imidazo[4,5-b]pyridinyl, imidazo[4,5-c]pyridinyl, pyrazolo[4,3-d]pyridinyl, pyrazolo[4,3-c]pyridinyl, pyrazolo[3,4-c]pyridinyl, pyrazolo[3,4-b]pyridinyl, isoindolyl, indazolyl, purinyl, indolininyl, imidazo[1,2-a]pyridinyl, imidazo[1,5-a]pyridinyl, pyrazolo[1,5-a]pyridinyl, pyrrolo[1,2-b]pyridazinyl, imidazo[1,2-c]pyrimidinyl, quinolinyl, isoquinolinyl, cinnolinyl, quinazolinyl, quinoxalinyl, phthalazinyl, pyrido[3,2-d]pyrimidinyl, pyrido[4,3-d]pyrimidinyl , pyrido[3,4-d]pyrimidinyl, pyrido[2,3-d]pyrimidinyl, pyrido[2,3-b]pyrazinyl, pyrido[3,4-b]pyrazinyl, pyrimido[5,4-d]pyrimidinyl, pyrazino[2,3-b]pyrazinyl and pyrimido[4,5-d]pyrimidinyl.

The term "heterocycloalkoxy" group as used in the present application comprises the heterocycloalkyl group that is bonded via an O atom to the rest of the molecule in question. Preferably the heterocycloalkoxy group is selected from the group of aziridinyl, azetidinyl, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, 1,1-dioxo-thiomorpholinyl, 12-crown-4, 15-crown-5, 18-crown-6, 21-crown-7, aza-18-crown-6, diaza-18-crown-6, aza-21-crown-7, and diaza-21-crown-7, preferably dioxolanyl, decahydroisoquinolyl, imidazolinyl, imidazolidinyl, isothiazolidinyl, isoxazolidinyl, morpholinyl, octahydroindolyl, octahydroisoindolyl, oxazolidinyl, piperidinyl, piperazinyl, 4-piperidonyl, pyrrolidinyl, tetrahydrofuryl, tetrahydropyranyl.

The term "heteroaryloxy" group as used in the present application comprises the heteroaryl group that is bonded via an O atom to the rest of the molecule in question. Preferabyl the heteroaryloxy group is selected from the group of heteroaryl groups selected from the group of pyrrolyl (2-pyrrolyl), pyrazolyl (3-pyrazolyl), imidazolyl (1-imidazolyl, 2-imidazolyl, 4-imidazolyl, 5-imidazolyl), triazolyl (1,2,3-triazol-1-yl, 1,2,3-triazol-2-yl 1,2,3-triazol-4-yl, 1,2,4- triazol-3-yl), oxazolyl (2-oxazolyl, 4-oxazolyl, 5-oxazolyl), isoxazolyl (3-isoxazolyl, 4-isoxazolyl, 5-isoxazolyl), thiazolyl (2-thiazolyl, 4-thiazolyl, 5-thiazolyl), thiophenyl (2-thiophenyl, 3-thiophenyl, 4-thiophenyl, 5-thiophenyl), furanyl (2-furanyl, 3-furanyl, 4-furanyl, 5-furanyl), pyridyl (2-pyridyl, 3-pyridyl, 4-pyridyl, 5-pyridyl), 5-tetrazolyl, pyrimidinyl (2-pyrimidinyl, 4-pyrimidinyl, 5-pyrimidinyl, 6-pyrimidinyl), pyrazinyl, pyridazinyl (3-pyridazinyl, 4-pyridazinyl, 5-pyridazinyl), quinolyl (2-quinolyl, 3-quinolyl, 4-quinolyl, 5-quinolyl, 6-quinolyl, 7-quinolyl, 8-quinolyl), isoquinolyl (1-isoquinolyl, 3-isoquinolyl, 4- isoquinolyl, 5-isoquinolyl, 6-isoquinolyl, 7-isoquinolyl, 8-isoquinolyl), benzo[b]furanyl (2-benzo[b]furanyl, 3-benzo[b]fura- nyl, 4-benzo[b]furanyl, 5-benzo[b]furanyl, 6-benzo[b]furanyl, 7-benzo[b]furanyl), 2,3-dihydro-benzo[b]furanyl (2-(2,3-di_hydrobenzo[b]furanyl), 3-(2,3-dihydro-benzo[b]furanyl), 4-(2,3-dihydro-benzo[b]furanyl), 5-(2,3-dihydrobenzo-[b]furanyl), 6-(2,3-dihydro-benzo-[b]furanyl), 7-(2,3-dihydro-benzo[b]furanyl)), benzo[b]thiophenyl (2-benzo[b]thiophenyl, 3- benzo[b]thiophenyl, 4-benzo[b]thiophenyl, 5-benzo[b]thiophenyl, 6-benzo[b]thiophenyl, 7-benzo[b]thiophenyl), 2,3-di_hydrobenzo[b]thiophenyl (2-(2,3-dihydro-benzo[b]thiophenyl), 3-(2,3-dihydro-benzo[b]thiophenyl), 4-(2,3-dihydrobenzo[b]thiophenyl), 5-(2,3-dihydro-benzo[b]thiophenyl), 6-(2,3-dihydrobenzo[b]thiophenyl), 7-(2,3-dihydro-benzo[b]thi_ophenyl)), 4,5,6,7-tetrahydrobenzo[b]thiophenyl (2-(4,5,6,7-tetrahydro-benzo[b]thiophenyl), 3-(4,5,6,7-tetrahydrobenzo[b]thiophenyl), 4-(4,5,6,7-tetrahydro-benzo[b]thiophenyl), 5-(4,5,6,7-tetrahydrobenzo[b]thiophenyl), 6-(4,5,6,7-tetrahydro-benzo[b]thiophenyl), 7-(4,5,6,7-tetrahydrobenzo[b]thiophenyl)), thieno[2,3-b]thiophenyl, 4,5,6,7-tetrahydro_thieno[2,3-c]pyridyl (4-(4,5,6,7-tetrahydro-thieno[2,3-c]pyridyl), 5-4,5,6,7-tetrahydro-thieno[2,3-c]pyridyl), 6-(4,5,6,7-tet_rahydro-thieno[2,3-c]pyridyl), 7-(4,5,6,7-tetrahydro-thieno[2,3-c]pyridyl)), indolyl (1-indolyl, 2-indolyl, 3-ihdolyl, 4-indolyl, 5-indolyl, 6-indolyl, 7-indolyl), isoindolyl (1-isoindolyl, 2-isoindolyl, 3-isoindolyl, 4-isoindolyl, 5-isoindolyl, 6-isoindolyl, 7- isoindolyl), 1,3-dihydro-isoindolyl (1-(1,3-dihydro-isoindolyl), 2-(1,3-dihydroisoindolyl), 3-(1,3-dihydro-isoindolyl), 4-(1,3- dihydro-isoindolyl), 5-(1,3-dihydro-isoindolyl), 6-(1,3-dihydro-isoindolyl), 7-(1,3-dihydro-isoindolyl)), indazole (1-inda_zolyl, 3-indazolyl, 4-indazolyl, 5-indazolyl, 6-indazolyl, 7-indazolyl), benzimidazolyl (1-benzimidazolyl, 2-benzimidazolyl, 4-benzimidazolyl, 5-benzimidazolyl, 6-benzimidazolyl, 7-benzimidazolyl, 8-benzimidazolyl), benzoxazolyl (1-benz-oxa_zolyl, 2-benzoxazolyl), benzothiazolyl (1-benzothiazolyl, 2-benzothiazolyl, 4-benzothiazolyl, 5-benzothiazolyl, 6-benzothiazolyl, 7-benzothiazolyl), benzo-[1,2,5]oxadiazolyl, (4-benzo[1,2,5]oxadiazole, 5-benzo[1,2,5]oxadiazole), carbazolyl (1-carbazolyl, 2-carbazolyl, 3-carbazolyl, 4-carbazolyl), piperidinyl (2-piperidinyl, 3-piperidinyl, 4-piperidinyl), pyrrolidinyl (1-pyrrolidinyl, 2-pyrrolidinyl, 3-pyrrolidinyl) that are bonded via an O atom to the rest of the molecule in question.

The term "alkylenheterocyclic" group used in the present application comprises a C₁-C₄₀ atoms containing group that comprises a C₁-C₂₀ alkylen chain wherein one H- atom is substituted by an hetereocyclic group. The alkylen chain is preferably selected from the group of methylene, ethylene, n-propylene, isopropylen, n-butylen, sec-butylen and tert.- butylene. The hetereocyclic group is preferably selected from C₄-C₃₉ heterocycloalkyl groups and aromatic heterocyclic groups. Preferably the alkylenheterocyclic group is selected from the group of 4-Methylen-1,3-dioxolan, 3-methylen-oxetan, 2-methylene furan, 2-methylene tetrahydrofuran.

The term "alkylenheterocyclicalkylen" group used in the present application comprises either an aromatic heterocyclic or a saturated heterocycloalkyl group in the center which comprises two directly at the C₂-C₃₈ containing hetereocyclic group bonded C₁-C₁₀ containing substituted or unsubstituted alkylen groups. These two C₁-C₁₀ alkylen groups are independently selected from the group of methylene, ethylene, n-propylene, iso-propylen, n-butylene, sec-butylene and tert.butylene groups which can be substituted or unsubstituted. Both C₁-C₁₀ containing alkylen groups are the divalent bondages to further groups which can be identical or different. Preferably heterocyclic groups are selected from the group of tetrahydrofuran, tetrahydrothiophene, pyrrolidine, tetrahydropyran, tetrahydrothiopyran, piperidin, 1,3- or 1,4 -dioxolan, 1,3- or 1,4-dithian,1,3- or 1,4-piperazin, furan, thiophen, pyrrol, pyridine. Most preferably alkylenheterocyclicalkylen groups are selected from the group of 2,5-dimethylentetrahydrofuran, 2,5-dimethylenfuran.

The term "fluoroaryl" group used in the present application comprises with C₁-C₄ alkyl groups substituted or unsubstituted C₆-C₁₀ aryl groups where at least one hydrogen atom of the alkyl group or the aryl group, preferably more than one of the alkyl groups and/or the aryl group and at most preferred all hydrogen atoms of the alkyl groups and/or the aryl group are replaced by fluorine atoms. Preferably unsubstituted C₆-C₁₀ aryl groups as fluoroaryl groups are selected from the group of pentafluorophenyl, 4-trifluoromethylphenyl, 4-perfluoro-tert-butylphenyl.

The term "alkenyl" group used in the present application comprises C₂-C₄₀ linear hydrocarbons having one or more C-C double bonds can be cumulated, alternating or statistic. Preferably alkenyl groups are selected from the group of ethenyl (vinyl), allyl, 1-propenyl, buten-1-yl, buten-2-yl, buta-1,3- dienyl, buta-1,2-dienyl, butatrienyl, penten-1-yl, penten-2-yl, penta-1,2-dienyl, penta-1,3-dienyl and penta-1,4-dienyl. More preferred alkenyl groups are selected from the group of ethenyl (vinyl), allyl and 1-propenyl.

The term "alkynyl" group used in the present application comprises C₂ to C₄₀ linear hydrocarbons having one or more C-C triple bonds can be alternating or statistic. Preferably alkynyl groups are selected from the group of ethynyl, propynyl, butyn-1-yl, butyn-2-yl, buta-1,3-diynyl, pentyn-1-yl, pentyn-2-yl, hexyn-3-yl. More preferably alkynyl groups are selected from the group of ethynyl, propynyl, butyn-2-yl and hexyn-3-yl.

The term "(poly)ether" group used in the present application comprises mono- and polyether groups. Monoether groups are those groups where only one -O- atom is connected to two C atoms of different or identical organyl groups (C-groups) at each side. The C-groups at each side of these monoether groups are independently selected from the group of C₁-C₃₉ containing substituted or unsubstituted alkylen, substituted or unsubstituted alkyl, substituted or unsubstituted mono- or divalent aryl, substituted or unsubstituted mono- or divalent cycloalkyl, substituted or unsubstituted unsaturated mono- or divalent cycloalkyl and substituted or unsubstituted mono- or divalent heterocyclic groups. Polyether groups are those groups where at least three C-groups or more are interrupted after each C-group by one -O- atom. If all the C-groups in the mono- or polyether groups are identical, these groups are symmetric mono- or polyether groups. If the C-groups are different, than these are unsymmetric mono- or polyether groups. (Poly)ether groups are bonded with one C-atom of one C-group to a further group, which is either the -O- or -N- atom of the -O-Y-group or of the -NR²-Y group or the (poly)ether group is the bridging group Z between X¹ and X² of the -X¹-Z-X²- group, where two C-groups of the (poly)ether group are bonded each with one C-atom to the -O- and/or N- atom of the X¹ and X² group. The C-groups for the polyether groups are the same as those for the monoether groups.

Preferably (poly)ether groups are those with 2 to 40 C- atoms overall included C-groups. Preferably symmetric monoether groups are selected from the group of methoxymethylen, methoxyoligo(methylol) (CH₃-[O-CH₂]_{w}-), ethoxyethylen, n-propoxy-n-propylen, n-butoxy-n-butylen and phenoxy-ethylen. Preferably symmetric polyether groups are selected from the group of (-CH₂-CH₂-O-)_{w}-CH₂-CH₂-, (-CH₂-CH₂-CH₂-O-)_{w}-CH₂-CH₂-CH₂-, (-C(CH₃)H-CH₂-O-)_{w}-C(CH₃)H-CH₂-, (-CH₂-C(CH₃)H-O-)_{w}-CH₂-C(CH₃)H-, (-C(CH₃)₂-CH₂-O-)_{w}-C(CH₃)₂-CH₂-, (-CH₂-CH(CH₃)-O-)_{w}-CH(CH₃)-CH₂-, (-CH₂-CH₂-CH₂-CH₂-O-)_{w}-CH₂-CH₂-CH₂-CH₂-, (-C(CH₃)H-CH₂-CH₂-O-)_{w}-C(CH₃)H-CH₂-CH₂-, (-CH₂-C(CH₃)H-CH₂-O-)_{w}-CH₂-C(CH₃)H-CH₂-, (-CH₂-CH₂-C(CH₃)H-O-)_{w}-CH₂-CH₂-C(CH₃)H-CH₂-, (-C(CH₃)₂-CH₂-O-)_{w}-C(CH₃)₂-CH₂-, (-C(CH₃)H-C(CH₃)H-O-)_{w}-C(CH₃)H-C(CH₃)H- and (-CH₂-C(CH₃)₂-O-)_{w}-CH₂-C(CH₃)₂- with w which can be any natural number from 2 to 38.
Preferably unsymmetric monoether groups are selected from the group of methoxyethylen, methoxy-n-propylen, methyoxy-isoproylen, methoxy-n-butylen, methoxy-isobutylen, methoxytert.-butylen, ethoxymethylen, ethoxy-n-propylen, ethoxy-isopropylen, ethoxybutylen, ethoxyisobutylen, ethoxy-tert.-butylen, n-propoxymethylen, n-propoxyethylen, n-propoxy-isoproylen, n-propoxy-n-butylen, n-propoxy-isobutylen, n-propoxy-tert.-butylen, isopropoxymethylen, isopropoxyethylen, isopropoxy-n-propylen, isopropoxy-n-butylen, isopropoxy-isobutylen, isoproxytert.-butylen, cyclopentanoxyethylen cyclohexanoxyethylen, phenoxyethylen, dicyclopentanyloxyethylen and dicyclopentenyloxyethylen. More preferred unsymmetric monoether groups are selected from the group of methoxyethylen, ethoxybutylen, cyclopentaoxyethylen, cyclohexanoxyethylen, phenoxyethylen, dicyclopentanyloxyethylen and dicyclopentenyloxyethylen. Preferably unsymmetric (poly)ether groups are selected from the group of C₁-C₃₉-alkylen-O-C₁-C₃₉-alkylen, C₁-C₃₉-alkylen-O-C₃-C₃₉-cycloalkyl, C₁-C₃₇-alkylen-O-C₃-C₃₉-unsaturated cycloalkyl, C₁-C₃₄-alkylen-O-C₆-C₃₉-aryl, C₁-C₃₈-alkylen-O-C₂-C₂₀-heterocyclic group, C₃-C₃₇-cycloalkyl-O-C₃-C₃₇-cycloalkyl, C₃-C₃₇-cycloalkyl-O-C₃-C₃₇-unsaturated cycloalkyl, C₃-C₃₄₄-cycloalkyl-O-C₆-C₃₇-aryl, C₃-C₃₈-cycloalkyl-O-C₂-C₂₀-heterocyclic group, C₃-C₃₇-unsaturated cycloalkyl-O-C₃-C₃₇-unsaturated cycloalkyl, C₃-C₃₄-unsaturated cycloalkyl-O-C₆-C₃₄ aryl, C₃-C₃₈-unsaturated cycloalkyl-O-C₂-C₂₀-heterocyclic group, C₆-C₄₃-aryl-O-C₆-C₃₄-aryl, C₆-C₃₈-aryl-O-C₂-C₂₀-heterocyclic group, C₂-C₂₀-heterocyclic group-O-C₂-C₂₀-heterocyclic group, wherein the number of C-atoms in both C-containing groups of each side of the -O- group are different, if the C-containing groups belong to the same kind of C-containing groups. More preferably unsymmetric (poly)ether groups are selected from the group of, C₁-C₃₉-alkylen-O-C₁-C₃₉-alkylen, C₁-C₃₇-alkylen-O-C₃-C₃₉-cycloalkyl, C₁-C₃₉-alkylen-O-C₁-C₃₉-alkylen and C₁-C₃₇-alkylen-O-C₃-C₃₉-cycloalkyl, wherein the number of C-atoms in both C-containing groups of each side of the -O-group are different, if the C-containing groups belong to the same kind of C-containing groups. The term "alkyloxy" group used in the present application is a special kind of mono- or polyether group and means an alkylen group that ends with an -O-atom. This -O-atom is, in contrast to the (poly)ether groups, bonded to a further group, which is the C-atom of the carbonyl group (-C(O)-) of E in formula (I). With the -C-atom of its alkylen group the alkyloxy group is bonded either to the -O-atom of a further (same or different) alkyloxy group, to a divalent bonded -O-atom or to a C-atom of the hydrocarbon chain that is substituted by the alkyloxy group. Preferably alkyloxy groups are C₁-C₂₀- alkyloxy groups. More preferably are those alkyloxy groups that are selected from the group of oxymethylen (-CH₂-O-), oxyethylen (-CH₂-CH₂-O-), oxy-n-propylen (-CH₂-CH₂-CH₂-O-), oxy-iso-propylen (CH₂-CH(CH₃)-O-), oxy-n-butylen (-CH₂-CH₂-CH₂-CH₂-O-), oxy-n-pentylen (-CH₂-CH₂-CH₂-CH₂-CH₂-O-), 2-(2-ethoxyethoxy)ethanol (ethyldiglycerol) (CH₃-CH₂-O-[CH₂-CH₂-O-]₂-), 2-(2-butoxyethoxy)ethanol (butyldiglycerol) (CH₃-CH₂-CH₂-CH₂-O-[CH₂-CH₂-O-]₂-) and 2-(2-Methoxyoligoethoxy)ethanol (methoxyoligoethylenglycol) (CH₃-O-[CH₂-CH₂-O-]_{y}-) with y= a natural number starting from 3 to 10). Most preferably are oxymethylen (-CH₂-O-), oxyethylen (-CH₂-CH₂-O-), oxy-n-propylen (-CH₂-CH₂-CH₂-O-), oxy-isopropylen (CH₂-CH(CH₃)-O-), ethyldiglycerol (CH₃-CH₂-O-[CH₂-CH₂-O-]₂-), butyldiglycerol (CH₃-CH₂-CH₂-CH₂-O-[CH₂-CH₂-O-]₂-) and methoxyoligoalkyleneglycol (CH₃-O-[CH₂-CH₂-O-]_{y}-) with y= a natural number selected from the group of 3, 4, 5 and 6 .

The term "keto" group used in the present application comprises at least a C₃-C₁₉ comprising carbon chain that is interrupted by at least one or more >C=O (carbonyl) groups that are not terminal (-C-C(O)-C-) wherein the carbon chains on both sides of the carbonyl group are independently selected from the group of substituted or unsubstituted C₁-C₈ alkyl, substituted or unsubstituted C₆-C₁₀ cycloalkyl and substituted or unsubstituted C₆- C₈ aryl groups. Preferably keto groups are selected from the group of methylketon (CH₃-C(O)-C-), ethylketon (CH₃-CH₂-C(O)-C-), propylketon (CH₃-CH₂-CH₂-C(O)-C-), butylketon (CH₃-CH₂-CH₂-CH₂-CH₂-C(O)-C), pentylketon (CH₃-CH₂-CH₂-CH₂-CH₂-C(O)-C-), acetoacetoxy (CH₃-C(O)-CH₂-C(O)-), (CH₃-C(O)-CH₂-C(O)-O-CH₂-CH₂-) to be derived from acetoacetoxyethylmethacrylate (AAEM) , (CH₃-C(O)-CH₂-C(CH₃)₂-) to be derived from diacetone acrylamide (DAAM), (H₃C-C(O)-CH₂-CH₂-C(O)-) to be derived from levulinic acid and CH₃-C(O)-CH₂-CH₂- to derived from methylvinyl ketone. More preferably keto groups are selected from the group of methylketon (CH₃-C(O)-C-), ethylketon (CH₃-CH₂-C(O)-C-), (CH₃-C(O)-CH₂-C(O)-), (CH₃-C(O)-CH₂-C(O)-O-CH₂-CH₂-) to be derived from acetoacetoxyethylmethacrylate (AAEM), (CH₃-C(O)-CH₂-C(CH₃)₂-) to be derived from diacetone acrylamide (DAAM), (H₃C-C(O)-CH₂-CH₂-C(O)-) to be derived from levulinic acid and CH₃-C(O)-CH₂-CH₂- to be derived from methylvinyl ketone.

The term "α,β- unsaturated carbonyl" groups used in the present application comprises C₃-C₄₀ atoms and at least one C-C double bond in directly neighborhood to a >C=O (carbonyl group) which can be an aldehyde carbonyl group, an keto carbonyl group, an acid carbonyl group, ester carbonyl, an imide carbonyl group or an amide carbonyl group. Preferably α,β- unsaturated carbonyl group are selected from the group of α,β- unsaturated ester carbonyl (H₂C=CH-C(O)-O~), (H₂C=C(CH₃)-C(O)-O~), (H₃C-HC=CH₂-C(O)-O~), HOOC-HC=CH₂-C(O)-O~, HOOC-C(CH₃)=CH-C(O)-O~, HOOC-CH=C(CH₃)-C(O)-O~, HOOC-CH₂-C(=CH₂)-C(O)-O~, α,β- unsaturated imide carbonyl group (maleimide or H₂C=CH-C(O)-NR-C(O)-C~) and α,β- unsaturated amid carbonyl groups (HzC=CH-C(O)-N<, H₂C=C(CH₃)-C(O)-N<). More preferably is the α,β-unsaturated carbonyl group selected from the group of (H₂C=CH-C(O)-O~), (H₂C=C(CH₃)-C(O)-O~), (H₃C-HC=CH₂-C(O)-O~), HOOC-HC=CH₂-C(O)-O~, HOOC-C(CH₃)=CH-C(O)-O~, HOOC-CH=C(CH₃)-C(O)-O~, HOOC-CH=C(CH₃)-C(O)-O~, HOOC-CH₂-C(=CH₂)-C(O)-O~ and H₂C=CH-C(O)-N<.

The term "substituted or unsubstituted" used in present application means that the described group can carry instead of one or more than one H-atoms, no, one or more substituents which are independently selected from the group of -OH, -CN, -C(O)N, -F, C₁-C₆ alkyl groups, C₁-C₆-alkylhydroxide groups, C₃-C₆ cycloalkyl groups, C₄-C₁₀ alkylencycloalkyl groups, C₃-C₆ unsaturated cycloalkyl groups, C₅-C₆ aryl groups, C₇- C₁₀ alkylenaryl groups, heterocyclic groups con-taining 3 to 5 carbon atoms and one or more heteroatoms selected from the group of N, O and S as ring members, (poly)ether groups containing C₂-C₂₀ carbon atoms, C₁-C₁₈ alkyloxy group, keto groups containing C₂-C₅ carbon atoms, α,β-unsaturated carbonyl groups containing C₃-C₃₀ carbon atoms, C₂-C₆ alkenyl groups, C₂-C₆ alkynyl groups and -NR⁸R⁹ groups, wherein R⁸ and R⁹ are independently selected from the group of H, C₁-C₆ alkyl groups, C₃-C₆- cycloalkyl groups, and C₅ -C₆ aryl groups.

The term "divalent" used in the present application comprising the ability of a group to be bonded to two different or same groups. These divalent groups were used as bridging groups which connect two parts of the molecule which are different or the same. These connections will be built at both ends of the bridging group and result to a longer chain or connect two other parts of the molecule via ring closure. The atoms of the divalent group that will be used for the connection to the other parts of the molecule are preferably independently selected from -CH₂-, two >CH-groups of a C₃-C₆ cycloalkyl group, two >CH-groups of a C₃-C₆ heterocycloalkyl group, a-CH₂-group of each alkyl group of a C₈-C₄₀ alkylenarylalkylen group, a -CH- group of each aryl group of a C₁₃-C₄₀ arylalkylenaryl groups.

Preferably OH substituted heterocyloalkyl group is 5-Hydroxy-1,3-dioxan.
Preferably alkylhydroxid substituted heterocycloalkyl groups are selected from the group of (2-Methyloltetrahydrofuran (tetrahydrofurfuryl) and 4-Methyol-1,3-dioxolan.

Preferabyl alky and alkyloxy substituted heterocycloalkyl groups are selected from the group of 5-Ethyl-5-methylol- 1,3-dioxan, 3-Ethyl-3-methyloloxetan and 2,2-Dimethyl-4-methylol-1,3-dioxolan (solketal).
Preferably alky substituted heterocycloalkyl groups are selected from the group of 1,2,2,6,6,-Pentamethylpiperid-3-yl and 1,2,2,6,6,-Pentamethylpiperid-4-yl.
Preferably alkyl and alkenyl substituted unsaturated cycloalkyl groups is 4-(isoprop-2-yl)-1-methylcyclohexene.
Preferably alkyl substituted cycloalkyl groups are selected from the group of 3,3,5-Trimethylcyclohexanyl, 4-tert.-Butylcyclohexanyl and 1,1,3,3-Tetramethylcyclobutan-2,4-diyl. Preferabyl NR⁸R⁹ substituted aryl group is dimethylaminopropyl.
Preferably -NCO substituted alkyl group is 2-isocyanatoethyl.
Preferably -CN substituted alkyl group is cyanatoethyl.
Preferably -F substituted alkyl groups are selected from the group of pentadecyfluorooctyl and trifluormethyl.
Preferably unsaturated cycloalkyl substituted alkylen groups is 2[4-Methylcylcohex-3-en-1-yl]propance-2-yl
Preferably -NR⁸R⁹ substituted aryl groups for the R¹⁰ group substituted with R^{10a} group are selected from the group of 2'-(Di-C₁-C₈ alkylamino)biphen-2-yl and 2-Di-C₁-C₈ alkylaminophen-1-yl, more preferably selected from the group of 2'-(N,N-dimethylamino)biphen-2-yl and 2-N,N-dimethylaminophen-1-yl groups.
Preferably aryl substituted heteroaromatic groups for the R¹⁰ group substituted with R^{10a} group are selected from the group of 1-phenylpyroll-2-yl and 1-phenylindol-2-yl.

In the inventive functionalized cyclic carbonates of formula (I) R¹ is either H or CH₃ and n is a natural number from 1 to 10, the E-group is selected from the group of -CH₂-CH₂-, -CH₂-CH₂-C(O)- and 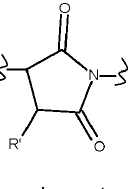 with R' which is selected from the group of H and a C₁-C₈ alkyl group, wherein the carbon atom of the terminal -CH₂- or the -CH- group next to the -CHR'- group of E is bonded to the -O- of formula (I), Q dependents on E and n and divides the inventive functionalized cyclic carbonates of formula (I) into inventive functionalized cyclic carbonates with special functional groups selected from the group of monovalent nitrile, monovalent oxazoline, divalent sulfone, divalent benzobisoxazole, monovalent acids, mono-, di- or multivalent esters and mono-, di or trivalent amides wherein the mono- or diester as special embodiment of the ester

group is a bisphenol A- compound and the mono- or divalent amides as special embodiment of the amide group is a mono- or divalent maleimides.

Preferred are the functionalized cyclic carbonates of formula (I) wherein E is selected from the group of -CH₂-CH₂- and -CH₂-CH₂-C(O)-, so that the inventive functionalized cyclic carbonates of formula (I) carry special functional groups selected from the group of monovalent nitrile, monovalent acids, mono-, di- or multivalent esters, mono-, di- or trivalent amides, monovalent oxazoline, divalent sulfones and divalent benzobisoxazole wherein n is preferably selected from the group of 1, 2, 3, 4 and 6.
More preferred are the inventive functionalized cyclic carbonates of formula (I) wherein E is - CH₂-CH₂-C(O)- and n is 1 or 2 so that the inventive alkynyl ether compounds of formula (I) carry special functional groups selected from the group of mono- and divalent esters and mono- or divalent amides.

If n =1 or 2 the mono and divalent inventive functionalized cyclic carbonates of formula (I) are received.
If n=1 and the E-group is ethylene (-CH₂-CH₂-), Q is selected from the group of -CN, and substituted or unsubstituted 2-oxazolin 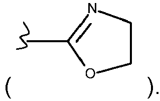 More preferred for n=1, E= -CH₂-CH₂- are the inventive functionalized cyclic carbonates selected from the group of 3-(2-(2-oxo-1,3-dioxolan-4-ylidene)ethoxy)propanenitrile (E and Z), 3-((1-(5-methyl-2-oxo-1,3-dioxolan-4-ylidene)propan-2-yl)oxy)propanenitrile (E and Z) with C₁- C₄ alkyl or C₁-C₄ alkylhydroxy groups substituted 4-(2-(2-(4,5-dihydrooxazol-2-yl)ethoxy)ethylidene)-1,3-dioxolan-2-one (E and Z) and with C₁- C₄ alkyl or C₁-C₄ alkylhydroxy groups substituted 4-(2-(2-(4,5-dihydrooxazol-2-yl)ethoxy)propylidene)-5-methyl-1,3-dioxolan-2-one (E and Z). Much more preferred for n=1 and E= - CH₂-CH₂- are the inventive functionalized cyclic carbonates selected from the group of 3-(2-(2-oxo-1,3-dioxolan-4-ylidene)ethoxy)propanenitrile (E and Z), 3-((1-(5-methyl-2-oxo-1,3-dioxolan-4-ylidene)propan-2-yl)oxy)propanenitrile (E and Z), 4-(2-(2-(4-methyl-4,5-dihydrooxazol-2-yl)ethoxy)ethylidene)-1,3-dioxolan-2-one (E and Z), 4-methyl-5-(2-(2-(4-methyl-4,5-dihydrooxazol-2-yl)ethoxy)propylidene)-1,3-dioxolan-2-one (E and Z), 4-(2-(2-(4,4-dimethyl-4,5-dihydrooxazol-2-yl)ethoxy)ethylidene)-1,3-dioxolan-2-one (E and Z), 4-(2-(2-(4,4-dimethyl-4,5-dihydrooxazol-2-yl)ethoxy)propylidene)-5-methyl-1,3-dioxolan-2-one (E and Z).

If n=2 and the E-group is ethylene (-CH₂-CH₂-), Q is selected from the group of a divalent sulfones, substituted or unsubstituted 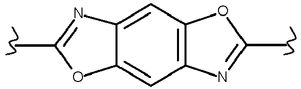 group and a divalent substituted or unsubstituted 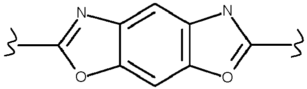 (benzobisoxazol-2,6-diyl) group. Preferred is Q selected from the group of divalent sulfones, unsubstituted divalent 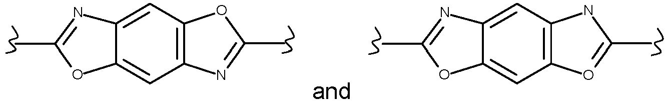 (benzobisoxazol-2,6-diyl) groups, if n=2 and E is ethylene (-CH₂-CH₂-). More preferred Q is selected from the group of (4Z,4'Z)-4,4'-(((benzo[1,2-d:5,4-d]bis(oxazole)-2,6-diylbis(ethane-2,1-diyl))bis(oxy))bis(ethan-2-yl-1-ylidene))bis(1,3-dioxolan-2-one), (4*Z*,4'*Z*)-4,4'-(((benzo[1,2-*d*:4,5-*d'*]bis(oxazole)-2,6-diylbis(ethane-2,1-diyl))bis(oxy))bis(ethan-2-yl-1-ylidene))bis(1,3-dioxolan-2-one), (5Z,5'Z)-5,5'-(((benzo[1,2-d:4,5-d]bis(oxazole)-2,6-diylbis(ethane-2,1-diyl))bis(oxy))bis(propan-2-yl-1-ylidene))bis(4-methyl-1,3-dioxolan-2-one), (5*Z*,5'*Z*)-5,5'-(((benzo[1,2-*d*:5,4-*d'*]bis(oxazole)-2,6-diylbis(ethane-2,1-diyl))bis(oxy))bis(propan-2-yl-1-ylidene))bis(4-methyl-1,3-dioxolan-2-one), (4Z,4'Z)-4,4'-(((sulfonylbis(ethane-2,1-diyl))bis(oxy))bis(ethan-2-yl-1-ylidene))bis(1,3-dioxolan-2-one) and (5*Z*,5'*Z*)-5,5'-(((sulfonylbis(ethane-2,1-diyl))bis(oxy))bis(propan-2-yl-1-ylidene))bis(4-methyl-1,3-dioxolan-2-one), if n = 2 and E is ethylene (-CH₂-CH₂-).

If n=1 or 2 and the E-group is 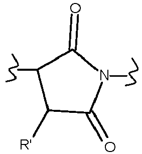 with R' which is selected from the group of H and a C₁-C₈ alkyl group, wherein the carbon atom of the terminal -CH- group next to the -CHR'- group of E is bonded to the -O- of formula (I) so that the maleimide group of E is a special embodiment of the inventive functionalized cyclic carbonates with an amide group as special functional group, Q is selected from the group of substituted or unsubstituted C₆-C₄₀ aryl groups, for n=1 and for n=2 Q is selected from the group of divalently bonded substituted or unsubstituted C₁₃-C₄₀ arylalkylenaryl groups and divalently bonded substituted or unsubstituted C₈-C₄₀ alkylenarylalkylen groups. Preferred is Q phenyl for n=1 and 1,3-Benzenedimethylen or bis (1,4-phenylen)methane and bis (2-Ethyl-5-metyl-1,4-phenylen)methane for n=2 and E =

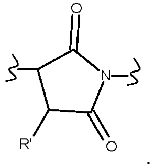

If n=1 or 2 and the E-group is -CH₂-CH₂-C(O)-, Q is selected from the group of -O-Y, -NR²-Y, as a special variation of NR²-Y the 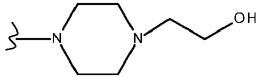 (4-(2-hydroxyethyl)piperazin-1-yl group, -O-Z-O-, -NR²-Z-NR²-, NR²-Z-O- and 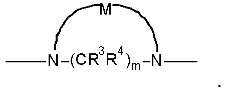 These inventive functionalized cyclic carbonates carry either an acid, an ester or an amide functional group.
If Q is the -O-Y or the -O-Z-O- group for n=1 or 2 and E = -CH₂-CH₂-C(O)- the inventive functionalized cyclic carbonates carry as special functional group an ester group.

If Q is selected from the group of -NR²-Y group, the 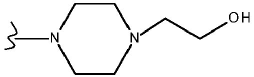 (4-(2-hydroxyethyl)piperazin-1-yl group , -NR²-Z-NR²-, -NR²-Z-O- and 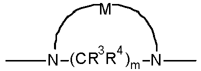 for n=1 or 2 and E = -CH₂-CH₂-C(O)-, the inventive functionalized cyclic carbonates carry as special functional group an amide group.
If Q is the -O-Y group and Y is H the functionalized cyclic carbonates carry as special functional group an acid group. The resulting acids are selected from the group of (E/Z)-3-(2-(2-oxo-1,3-dioxolan-4-ylidene)ethoxy)propanoic acid and (E/Z)-3((1-(5-methyl-2-oxo-1,3-dioxolan-4-ylidene)porpan-2-yl)oxy)propanoic acid.
As well for the esters as for the amides Y is selected from the group of substituted or unsubstituted C₁-C₄₀ alkyl groups, substituted or unsubstituted saturated or unsaturated C₃-C₄₀ cycloalkyl groups, substituted or unsubstituted C₂-C₄₀ (poly)ether groups, substituted or unsubstituted saturated or unsaturated heterocyclic groups containing 2 to 20 carbon atoms and one or more heteroatoms selected from the group of N, O, and S as ring members, substituted or unsubstituted saturated or unsaturated alkylenheterocyclic groups containing 3 to 20 carbon atoms and one or more heteroatoms selected from the group of N, O, and S as ring members, substituted or unsubstituted C₆-C₄₀ aryl groups, substituted or unsubstituted C₂-C₄₀ alkenyl groups, substituted or unsubstituted C₂-C₄₀ alkynyl groups, substituted or unsubstituted saturated or unsaturated C₃-C₄₀ alkylencycloalkyl groups, substituted or unsubstituted C₇-C₄₀ alkylenaryl groups, substituted or unsubstituted α,β- unsaturated carbonyl groups containing C₃-C₄₀ atoms, substituted or unsubstituted C₂-C₅ keto groups and substituted or unsubstituted C₁-C₄ alkylen group which is part of a substituted or unsubstituted saturated heterocyclic group that is build up between R² of the -NR²- group and Y to form

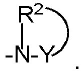

As well for the esters as for the amides Z is selected from the group of substituted or unsubstituted C₁-C₄₀ alkylen groups, divalently substituted or unsubstituted C₂-C₄₀ (poly)ether groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded substituted or unsubstituted alkylenheterocyclicalkylen groups containing 4 to 20 carbon atoms and one or more heteroatoms selected from the group of N, O, and S as ring members, divalently bonded substituted or unsubstituted C₆-C₄₀ aryl groups, divalently bonded substituted or unsubstituted C₇-C₄₀ cycloalkylalkylencycloalkyl groups, divalently bonded substituted or unsubstituted saturated or unsaturated C₅-C-₄₀ alkylencycloalkylalkylen groups, divalently bonded substituted or unsubstituted C₁₃-C₄₀ arylalkylenaryl groups, divalently bonded substituted or unsubstituted C₈-C₄₀ alkylenarylalkylen groups and divalently bonded substituted or unsubstituted C₁₇-C₃₀ alkylen-bisphenol A -alkylen groups.

In 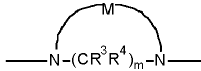 group the groups R³ and R⁴ are independently selected from the group of H and substituted or unsubstituted C₁-C₄₀ alkyl groups, while m is a natural number selected from the group of 1, 2 or 3 and M is selected from the group of substituted or unsubstituted C₁-C₃ alkylen groups, divalently bonded -NR⁵-CR³R⁴- groups, divalently bonded -NR⁵-NR⁶- groups, divalently bonded -NR⁵-CR³R⁴-CR³R⁴- groups, divalently bonded -O-CR³R⁴-CR³R⁴- groups, divalently bonded -S-CR³R⁴-CR³R⁴- groups, divalently bonded CR³R⁴-O-CR³R⁴ groups, divalently bonded -CR³R⁴-S-CR³R⁴- groups and divalently bonded -CR³R⁴-NR⁵-CR³R⁴- groups with R⁵ and R⁶ are independently selected from the group of H, substituted or unsubstituted C₁-C₄₀ alkyl groups, C₆-C₄₀ aryl groups, C₃-C₄₀ cycloalkyl groups and -C(O)CH=CH₂ group.

Preferred inventive functionalized cyclic carbonates with an ester functional group are selected from the group where n=1 or 2 and E = -CH₂-CH₂-C(O)- and wherein Y in the -O-Y group is selected from the group of substituted or unsubstituted C₁-C₄₀ alkyl groups, substituted or unsubstituted saturated or unsaturated C₃-C₄₀ cycloalkyl groups, substituted or unsubstituted saturated alkylenheterocyclic groups containing 3 to 20 carbon atoms and one or more heteroatoms selected from the group of N, O, and S as ring members, unsubstituted, saturated C₃-C₄₀ alkylencycloalkyl groups and
Z which is selected from the group of substituted or unsubstituted C₁-C₄₀ alkylen groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded saturated or unsaturated substituted or unsubstituted C₅-C₄₀ alkylencycloalkylalkylen groups and divalently bonded substituted or unsubstituted C₁₇-C₃₀ alkylen-bisphenol A- alkylen groups.
More preferred is Y in the -O-Y group for the inventive functionalized cyclic carbonates with an ester functional group for n=1 selected from the group of substituted or unsubstituted C₁-C₁₀ alkyl groups, substituted or unsubstituted saturated or unsaturated C₄-C₁₁ cycloalkyl groups, substituted or unsubstituted saturated alkylenheterocyclic groups containing 3 to 10 carbon atoms and one or more O-atoms as heteroatom and as ring members, unsubstituted, saturated C₈-C₁₂ alkylencycloalkyl groups. Most preferred is Y in the -O-Y group for the inventive functionalized cyclic carbonates with an ester functional group for n=1 selected from the group of tert.-butyl, n-propyl, 2,2-dimethylpropan-3-yl-1-ol, 2-hydroxylpropyl, isobornyl, bornyl, menthyl, ter-pinyl, dihydrodicyclopentadienyl, cyclopentenyl, cyclohexanyl, norbornenyl, 3,3,5-Trimethylcyclohexanyl, 4-tert.-Butylcyclohexyl, 4-methylen-1,3-dioxolan, 4-methylen-2,2,dimethyl-1,3-dioxolan and 3-methylen-3-ethyl-1-oxetan.
More preferred is Z in the -O-Z-O- group for the inventive functionalized cyclic carbonates with an ester functional group for n=2 selected from the group of substituted C₃-C₄₀ alkylen groups, divalently bonded substituted C₃-C₁₀ cycloalkyl groups, divalently bonded unsubstituted heterocyclic groups containing 3 to 10 carbon atoms and one or more -O-atoms as heteroatoms and as ring members, divalently bonded saturated unsubstituted C₅-C₁₄ alkylencycloalkylalkylen groups and divalently bonded unsubstituted C₁₇-C₂₅ alkylen-bisphenol A-alkylen groups. Most preferred is Z in the -O-Z-O- group for the inventive functionalized cyclic carbonates with an ester functional group for n=2 selected from the group of 2,2-dimethyl-propan-1,3-diyl, 2,2,4,4-Tetramethylcyclobutan-1,3-diyl, Tricyclo[5.2.1.0²⁶]decan-4,8-dimethylen, hexahydro-furo[3,2-b]furan-3,6-yl and Bisphenol A -bis(2-hydroxypropy-3-diyl)ether.

Preferred inventive functionalized cyclic carbonates with an amide functional group are selected from the group where n=1 or 2 and E = -CH₂-CH₂-C(O)- and wherein Y in the -NR²-Y group is selected from the group of substituted or unsubstituted C₁-C₄₀ alkyl groups, 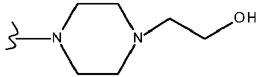 (4-(2-hydroxyethyl)piperazin-1-yl group and substituted or unsubstituted C₆-C₄₀ aryl groups with R² selected from the group of H and substituted or unsubstituted C₁-C₄₀ alkyl groups, and Z in the -NR²-Z-NR²- and -NR²-Z-O- group is selected from the group of substituted or unsubstituted C₁-C₄₀ alkylen groups, divalently bonded substituted or unsubstituted C₆-C₄₀ aryl groups and divalently bonded substituted or unsubstituted alkylenheterocyclicalkylen groups containing 4 to 20 carbon atoms and one or more heteroatoms selected from the group of N, O, and S as ring members,
and wherein in the 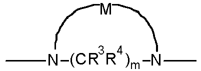 group R³ and R⁴ are independently selected from the group of H and substituted or unsubstituted C₁-C₁₀ alkyl groups, while m is a natural number selected from the group of 1 and 2 and M is selected from the group of substituted or unsubstituted C₁-C₃ alkylen groups and divalently bonded -CR³R⁴-NR⁵-CR³R⁴- groups with R⁵ and R⁶ are independently selected from the group of H, substituted or unsubstituted C₁-C₄₀ alkyl groups, C₆-C₄₀ aryl groups, C₃-C₄₀ cycloalkyl groups and -C(O)CH=CH₂ group.
More preferred is Y in the -NR²-Y group for the inventive functionalized cyclic carbonates with an amide functional group for n=1 selected from the group of OH-substituted C₂-C₅ alkyl group, keto- and alkyl-substituted C₃-C₁₀ alkyl group, OH-substituted C₆-C₄₀ aryl groups. Most preferred is Y in the -NR²-Y group for the inventive functionalized cyclic carbonates with an amide functional group for n=1 selected from the group of methylhydroxid, eth-2yl-hydroxid, 2-Methylpropan-2yl-3- ol, 2-Hydroxypropyl, 2-methoxy-1,1-dimethylethyl and p-Hydroxyphenyl, with R² selected from the group of H and 2-Hydroxypropyl.
More preferred is Z in the -NR²-Z-NR²- and -NR²-Z-O- group for the inventive functionalized cyclic carbonates with an amide functional group for n=2 selected from the group of unsubstituted C₁-C₄₀ alkylen groups, divalently bonded substituted or unsubstituted C₆-C₄₀ aryl groups and divalently bonded unsubstituted alkylenheterocyclicalkylen groups containing 4 to 20 carbon atoms and one or more heteroatoms selected from the group of N, O, and S as ring members. Most preferred is Z in the -NR²-Z-NR²- and -NR²-Z-O- group for the inventive functionalized cyclic carbonates with an amide functional group for n=2 selected from the group of methylen, ethylene, 1,3-phenyl, 2,5-Dihydroxy-1,4-phenyl, 2,5-dimethylentetrahydrofuran and 2,5-dimethylenfuran with R²= H.

More preferred is M = -CH₂-CH₂- and R³ and R⁴= H with m= 2 in the 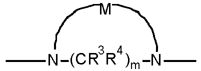 Group so that it is a 1,4-diazinanyl (piperazinyl) group.

Preferred inventive functionalized cyclic carbonates of formula (I) with multivalent ester or amide groups as special functional groups carry an -CH₂-CH₂-C(O)- group as E, n is selected from the group of 3 and ≥ 4 and Q is selected from the group of a substituted or unsubstituted 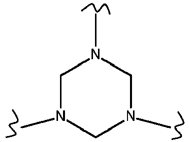 (1,3,5-triazinan) group, a 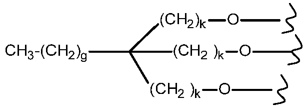 group, a 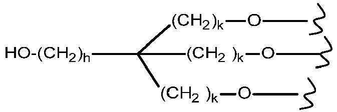 group, a 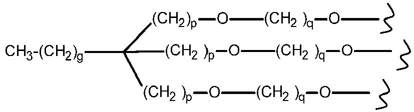 group, 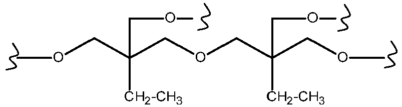 group and 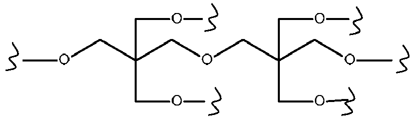 group, wherein
- g: is a natural number selected from 1 to 33,
- k: is a natural number selected from 1 to 12,
- h: is a natural number selected from 1 to 15,
- p: is a natural number selected from 1 to 3 and
- q: is a natural number selected from 1 to 5, wherein the sum of C-atoms in the groups with g, k, h, p and q is not higher than 40.

For n=3, Q is preferably selected from the group of with three -C(O)-CH₂-CH₂-O-CHR¹-C≡C-CHR¹-OH groups substituted 1,3,5-triazinan, the group where g= 1-4 and k = 1-3, the group where h =1-15 and k =1-3 and the group where g = 1-4, p = 1-3 and q =1-3. More preferably for n=3 Q is selected from the group of with three -C(O)-CH₂-CH₂-O-CHR¹-C≡C-CHR¹-OH groups substituted 1,3,5-triazinan, the group where g=1 and k=1, the group where h= 1, k=1, the group where g=1, p=1 and q=3.
For n≥ 4, preferably n = 4 or 6, Q is preferably selected from the group of

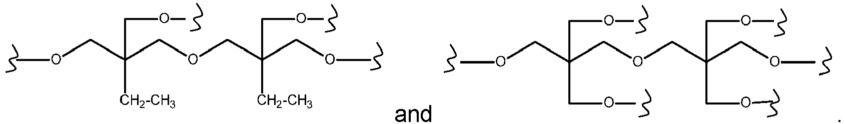

In order to produce the inventive cyclic carbonates of formula (I) the alkynyl ether compounds of formula (II) are reacted with carbon dioxide in the presence of at least one transition metal catalyst TMC1 which comprises Ag as transition metal and at least one bulky ligand selected from the group of compounds of formula (III) and compound of formula (IV)

The transition metal catalyst TMC1 of the process of the invention can be employed in the form of a preformed metal complex which comprises Ag and at least one bulky ligand selected from the group of ligands consisting of compounds of formula (III) or compounds of formula (IV), preferably compounds of formula (III), as shown above. Alternatively, the transition metal catalyst TMC1 is formed in situ in the reaction medium by combining an Ag compound, herein also termed pre-catalyst, which does not comprise any bulky ligand, with one or more suitable bulky ligand to form a catalytically active Ag complex, the transition metal catalyst TMC1, in the reaction medium. In case the bulky ligand is a N-heterocyclic carbene ligand (NHC-ligand) of formula (IV), it is also possible that the transition metal catalyst TMC1 is formed in situ in the reaction medium by combining a pre-catalyst with one or more NHC-precursor, in particular the protonated form of a NHC-ligand, which is in situ converted to the corresponding NHC-ligand of formula (IV), to form a catalytically active Ag complex in the reaction medium.

In one embodiment of the present invention, the inventive process is characterized in that the transition metal catalyst TMC1 is prepared in situ by using an Ag compound, which does not comprise any bulky ligand, the compound of formula (III) or formula (IV) as bulky ligand or the protonated form of the compound of formula (IV) represented by formula (V), 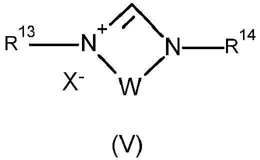 wherein R¹³, R¹⁴ and W are defined as described above and X⁻ is an anion equivalent, together with a base.

Suitable bases for deprotonating the protonated form of different NHC ligands according to formula (V) are described by de Frémont et al., Coordination Chemistry Reviews 253 (2009) 876 to 881. The deprotonation of the protonated forms of NHC ligands can be carried out in ammonia or in non-protic solvents such as THF or ethers. The deprotonation requires anhydrous conditions and the use of strong bases, with pKₐ values above 14. Usually, potassium or sodium hydride with a catalytic amount of tert-butoxide is employed, but tert-butoxide itself, lithium aluminum hydride, n-butyllithium, MeLi, t-BuLi, potassium hexamethyldisilazide (KHMDS) and 1,8-diazabicyclo[5.4.0]undec-7-ene (DBU) are also efficient alternatives.

Suitable pre-catalysts are selected from neutral metal complexes, oxides and salts of Ag. More preferred silver compounds that are useful as pre-catalyst are selected from the group of Ag(OAc), AgF, AgNO₃, silver trifluoroacetate, Ag₂O and Ag₂CO₃. Most preferred silver compounds, also called pre-catalyst, are selected from the group of AgOAc, AgF, Ag₂O and Ag₂CO₃.

In addition to the transition metal, the transition metal catalyst TMC1 comprises at least one bulky ligand selected from the group of ligands of compounds of formula (III) and compounds of formula (IV), preferably compounds of formula (III).

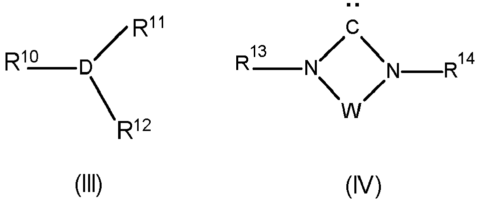

In case the bulky ligand is a compound of formula (III), 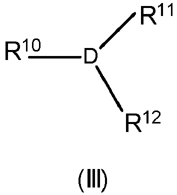 wherein
- D: is selected from the group of P, As or Sb, preferably P or As, in particular P,
- R¹⁰: is selected from the group of a substituted or unsubstituted C₃-C₄₀ cycloalkyl group, a substituted or unsubstituted C₂-C₄₀ heterocycloalkyl group, a substituted or unsubstituted C₆-C₄₀ aryl group, a substituted or unsubstituted C₂-C₄₀ heteroaromatic group, a substituted or unsubstituted C₃-C₄₀ cycloalkoxy group, a substituted or unsubstituted C₂-C₄₀ heterocycloalkoxy group, a substituted or unsubstituted C₆-C₄₀ aryloxy group and a substituted or unsubstituted C₂-C₄₀ heteroaryloxy group, in particular R¹⁰ is a substituted or unsubstituted C₃-C₄₀ cycloalkyl group, a substituted or unsubstituted C₂-C₄₀ heterocycloalkyl group, a substituted or unsubstituted C₆-C₄₀ aryl group or a substituted or unsubstituted C₂-C₄₀ heteroaromatic group, even more preferably R¹⁰ is a substituted or unsubstituted C₆ to C₄₀ aryl group or a substituted or unsubstituted C₂ to C₄₀ heteroaromatic group, if R¹⁰ is a substituted ring cyclus of the one mentioned above, R¹⁰ is preferably substituted in at least one of the two ortho position of the ring cyclus relative to D with a group R^{10a},
wherein R^{10a} is selected form the group of substituted or unsubstituted C₆-C₃₄ aryl group, substituted or unsubstituted C₁-C₁₀ alkoxy group, substituted or unsubstituted C₂-C₁₂ dialkylamino group and where R^{10a} forms with an adjacent group substituting R¹⁰ in the meta position of the ring cyclus together with the atoms connecting them a monocyclic or polycyclic, substituted or unsubstituted aliphatic, aromatic or heteroatom comprising ring system comprising 4 to 40 carbon atoms wherein the heteroatoms are selected from the group of Si, Ge, N, P, O and S, more preferably R^{10a} is selected from the group of methoxy, ethoxy, isopropoxy or cyclohexyloxy, dimethyl amino, diethyl amino, di-isopropyl amino, N-morpholinyl and N-piperidyl, and
R¹¹, R¹² are independently of each other selected from the group of substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₂₀ heterocycloalkyl groups, substituted or unsubstituted C₆-C₄₀ aryl groups, substituted or unsubstituted C₂-C₄₀- heteroaromatic groups, substituted or unsubstituted C₆-C₁₀ fluoroaryl groups, substituted or unsubstituted C₂-C₂₀ alkynyl groups and substituted or unsubstituted C₇-C₄₀- alkylenaryl groups, preferably R¹¹ and R¹² are independently of each other selected from the group of a substituted or unsubstited C₃-C₂₀ cycloalkyl, a substituted or unsubstituted C₂-C₂₀ heterocycloalkyl, a substituted or unsubstituted C₃-C₂₀ alkyl and substituted or unsubstituted C₆-C₁₄ aryl groups, more particular R¹¹ and R¹² are independently of each other selected from the group of tert.-butyl, adamantly, cyclohexyl and phenyl, much more particular R¹¹ and R¹² are identical.

In case the bulky ligand is a compound of formula (IV), 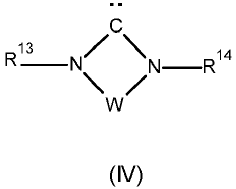 wherein
- R¹³ and R¹⁴: are independently of each other selected from the group of substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₄₀ cycloalkoxy group, substituted or unsubstituted C₃-C₂₀ heterocycloalkyl groups, a substituted or unsubstituted C₂-C₄₀ heterocycloalkoxy group, substituted or unsubstituted C₆-C₄₀ aryl groups, substituted or unsubstituted C₆-C₄₀ aryloxy group, substituted or unsubstituted C₂-C₄₀- heteroaromatic groups, substituted or unsubstituted C₆-C₁₀ fluoroaryl groups, substituted or unsubstituted C₂-C₂₀ alkynyl groups and substituted or unsubstituted C₇-C₄₀- alkylenaryl groups, preferably R¹³ and R¹⁴ are independently of each other selected from the group of substituted or unsubstituted C₁-C₄₀ alkyl groups, substituted or unsubstituted C₁-C₁₂ alkoxy groups, substituted or unsubstituted C₃-C₂₀ heterocycloalkyl groups, substituted or unsubstituted C₆-C₄₀ aryl groups, substituted or unsubstituted C₂-C₄₀- heteroaromatic groups, substituted or unsubstituted C₆-C₁₀ fluoroaryl groups, substituted or unsubstituted C₂-C₂₀ alkynyl groups and substituted or unsubstituted C₇-C₄₀- alkylenaryl groups wherein R¹³ and/or R¹⁴ is substituted in at least one of the two ortho positions relative to N with R^{13a} or R^{14a}, wherein R^{13a} and R^{14a} are independently of each other are a substituted or unsubstituted C₁-C₁₀ alkyl group more preferably an isopropyl group.
and
- W: is a divalent bridging group selected from -CR¹⁵=CR¹⁶-, -CR¹⁵=N-, -CR¹⁵R¹⁷-CR¹⁶R¹⁸- and -CR¹⁵R¹⁷-CR¹⁶R¹⁸-CR¹⁹R²⁰-, wherein
R¹⁵, R¹⁶ R¹⁷, R¹⁸, R¹⁹ and R²⁰ are independently from each other hydrogen, substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₄₀ cycloalkoxy group, substituted or unsubstituted C₃-C₂₀ heterocycloalkyl groups, substituted or unsubstituted C₂-C₄₀ heterocycloalkoxy group, substituted or unsubstituted C₆-C₄₀ aryl groups, substituted or unsubstituted C₆-C₄₀ aryloxy group, substituted or unsubstituted C₂-C₄₀- heteroaromatic groups, substituted or unsubstituted C₆-C₁₀ fluoroaryl groups, substituted or unsubstituted C₂-C₂₀ alkynyl groups, substituted or unsubstituted C₇-C₄₀- alkylenaryl groups and two adjacent groups R¹⁵ and R¹⁶ and/or R¹⁸ and R¹⁹ together with the atoms connecting them of the group W build up a monocyclic or polycyclic, substituted or unsubstituted, aliphatic, aromatic or heteroatom comprising carbon ring system containing 4 to 40 carbon atoms and wherein the heteroatom comprising carbon ring system comprises one or more heteroatoms selected from the group of Si, Ge, N, P, O and S as ring members, preferably R¹⁵, R¹⁶ R¹⁷, R¹⁸, R¹⁹ and R²⁰ are identical, more preferably R¹⁵, R¹⁶ R¹⁷, R¹⁸, R¹⁹ and R²⁰ are H.

In one embodiment of the present invention, the inventive process is characterized in that the bulky ligand is selected from a compound of formulas A' to P and mixtures thereof, preferably a compound of formulas A' to D and mixtures thereof.

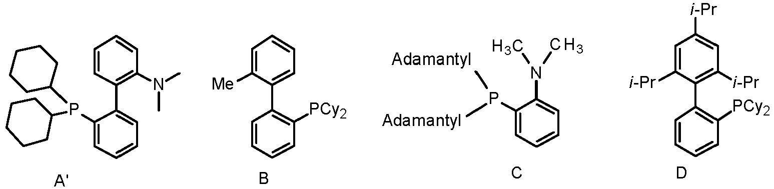

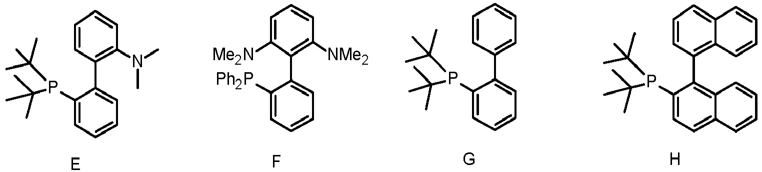

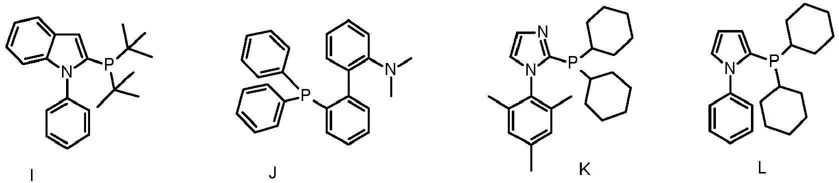

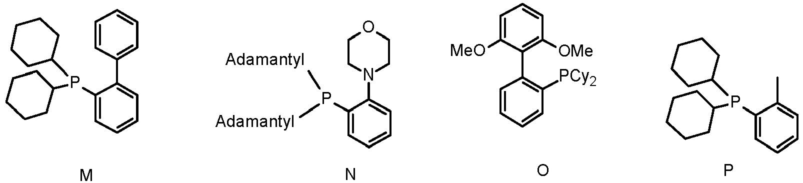

In one embodiment of the present invention, the inventive process is characterized in that the transition metal catalyst TMC1 is selected from a compound of the following formulas. 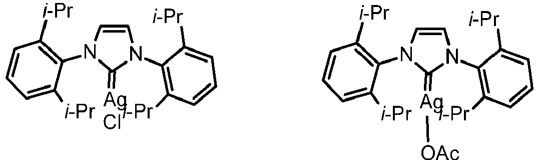 The molar ratio of the bulky ligand to the transition metal of transition metal catalyst TMC1 can be varied in wide range. Preferably the molar ratio of the bulky ligand to the transition metal is below 2. More preferably the ratio of the bulky ligand to the transition metal is in the range from 0.2 to 1.8, even more preferably in the range from 0.3 to 1.5, in particular in the range from 0.4 to 1.2.

In one embodiment of the present invention, the inventive process is characterized in that the molar ratio of the bulky ligand to the transition metal of transition metal catalyst TMC1 is in the range from 0.4 to 1.2.

In the inventive process the amount of transition metal catalyst TMC1 used in process step a) based on the amount of alkynyl ether compound of (II) can be varied in a wide range. Usually, the transition metal catalyst TMC1 is used in a sub-stoichiometric amount relative to the alkynyl ether compound of formula (II). Typically, the amount of transition metal catalyst TMC1 is not more than 50 mol%, frequently not more than 20 mol% and in particular not more than 10 mol% or not more than 5 mol%, based on the amount of alkynyl ether compound of formula (II). An amount of transition metal catalyst TMC1 of from 0.001 to 50 mol%, frequently from 0.001 mol% to 20 mol% and in particular from 0.005 to 5 mol%, based on the amount the alkynyl ether compound of formula (II) is preferably used in the process of the invention. Preference is given to using an amount of transition metal catalyst TMC1 of from 0.01 to 5 mol%. All amounts of transition metal complex catalyst indicated are calculated as transition metal and based on the amount of alkynyl ether compound of formula (II).

In one embodiment of the present invention, the inventive process is characterized in that the amount of transition metal catalyst TMC1 used in process step a) based on the amount of alkynyl ether compound of formula (II) is in the range from 0.005 to 5 mol%.

The CO₂ used for the carboxylation-cyclisation reaction can be used in pure form or, if desired, also in the form of mixtures with other, preferably inert gases, such as nitrogen or argon. Preference is given to using CO₂ in undiluted form.

The reaction is typically carried at a CO₂ pressure in the range from 0.1 to 200 bar, preferably in the range from 1 to 50 bar, more preferably in the range from 1 to 40 bar.

In one embodiment of the present invention, the inventive process is characterized in that the process step a) is performed at a pressure in the range from 1 to 50 bar, more preferably in the range from 1 to 40 bar.

The reaction can principally be performed continuously, semi-continuously or discontinuously. Preference is given to a continuous process.

The reaction can principally be performed in all reactors known by a person in the art for this type of reaction and therefore, will select the reactors accordingly. Suitable reactors are described and reviewed in the relevant prior art, e.g. appropriate monographs and reference works such as mentioned in US 6639114 B2, column 16, line 45-49. Preferably, for the reaction an autoclave is employed which may have an internal stirrer and an internal lining.

The composition obtained in the carboxylation-cyclisation reaction of the present invention comprises an unsubstituted exo-vinylene carbonate, that is a cyclic carbonate of formula (I).

Process step a) of the inventive process can be performed in a wide temperature range. Preferably process step a) is performed at a temperature in the range preferably in the range from 0 °C to 150 °C and particularly preferably in the range from 10°C to 80 °C. Temperatures below 100 °C have surprisingly been found to be particularly advantageous.

In one embodiment of the present invention, the inventive process is characterized in that the process step a) is performed at a temperature in the range from 0 °C to 100 °C, preferably in the range from 10°C to 80 °C.

The process of the invention can be carried out in the presence of a solvent. Suitable solvents are selected from aliphatic hydrocarbons, aromatic hydrocarbons, halogenated hydrocarbons, amides, ureas, nitriles, sulfoxides, sulfones, esters, carbonates, ethers, alcohols and mixtures thereof. Preferred solvents are
- aliphatic hydrocarbons such as pentane, hexane, heptane, octane or cyclohexane;
- aromatic hydrocarbons such as benzene, toluene, xylenes, ethylbenzene, mesitylene or benzotrifluoride;
- halogenated hydrocarbons such as dichloromethane,
- amides such as dimethylformamide, diethylformamide, N-methylpyrrolidone, N-ethylpyrrolidone or dimethylacetamide;
- ureas such as tetramethylurea, N,N-dimethylimidazolinone (DMI) and N,N-dimethylpropyleneurea (DMPU);
- nitriles such as acetonitrile or propionitrile;
- sulfoxides such as dimethyl sulfoxide;
- sulfones such as sulfolane;
- esters such as methyl acetate, ethyl acetate, t-butyl acetate;
- carbonates such as diethyl carbonate, ethylene carbonate and propylene carbonate; and
- ethers such as dioxane, tetrahydrofuran, diethyl ether, dibutyl ether, methyl t-butyl ether, diisopropyl ether or diethylene glycol dimethyl ether;

If desired, mixtures of two or more of the afore-mentioned solvents can also be used.

Preference is given to using dichloromethane, acetone, dimethylformamide or acetonitrile as solvents.

In one embodiment of the present invention, the inventive process is characterized in that the reaction is carried out in the presence of a solvent selected from aliphatic hydrocarbons, aromatic hydrocarbons, halogenated hydrocarbons, amides, ureas, nitriles, sulfoxides, sulfones, esters, carbonates, ethers, alcohols and mixtures thereof, preferably selected from dichloromethane, acetone, dimethylformamide or acetonitrile.

Alternatively, the process of the invention can be carried out in the absence of any of the above-mentioned organic solvent, so-called neat conditions, preferably in the presence of liquid or supercritical carbon dioxide, in particular in the presence of supercritical carbon dioxide.

Alternatively, the process of the invention can be carried out in the presence of liquid or supercritical carbon dioxide, in particular in the presence of supercritical carbon dioxide, which is mixed with one of the above-mentioned organic solvent, so-called CO₂-expanded solvents.

The composition obtained in the carboxylation-cyclization of the invention comprises an unsubstituted exo-vinylene carbonate, the cyclic carbonate of formula (I).

The work-up of the reaction mixture of the inventive process and the isolation of the cyclic carbonate of formula (I) is effected in a customary manner, for example by filtration, an aqueous extractive work-up or by a distillation, for example under reduced pressure. The cyclic carbonate of formula (I) may be obtained in sufficient purity by applying such measures or a combination thereof, obviating additional purification steps. Alternatively, further purification can be accomplished by methods commonly used in the art, such as chromatography.

In one embodiment of the present invention, the inventive process is characterized in that the cyclic carbonates of formula (I) are separated from the transition metal catalyst TMC1 after process step a) via distillation, extraction, precipitation and/or crystallization.

The distillation residue usually still comprises the transition metal catalyst TMC1 in an active form, that can be reused in a new carboxylation-cyclisation reaction step, that is a new process step a. As long as the distillation conditions, in particular the temperature treatment, are not too harsh, the transition metal catalyst TMC1 remains active.

In one embodiment of the present invention, the inventive process is characterized in that the transition metal catalyst TMC1 is recycled to the reaction step a) after the cyclic carbonate of formula (I) was removed via distillation, extraction, precipitation and/or crystallization.

The cyclic carbonates of formula (I), which are prepared according to the inventive process show a high purity and are advantageously used in applications such as monomer in polymerization reactions

A further aspect of the invention is the use of the cyclic carbonates of formula (I) prepared according to the above described inventive process as precursors in polymerization reactions with compounds comprising OH-group, primary amino and/or secondary amino groups.

The inventive process for preparing cyclic carbonates of formula (I) allows not only the preparation of known compounds (e.g. R² = H) but also the preparation of new compounds, which can be used as precursor of monomers in polymerization reactions or as building blocks in the synthesis of active compounds for pharmaceutical or agricultural applications.

### Examples

### experimental section

### Example 1

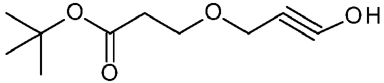

### Synthesis of the tert-Butyl-BYD adduct (tBuA-BYD) (tert.-Butyl-3-(4-hydroxybut-2-nyoxy)propanoate) in solution

2-Butyne-1,4-diol (537 g, 6.24 mol, 10.0 eq.) was placed in a 2 L three-neck flask and dissolved in 600 mL 1,4-dioxane. The mixture was heated to 60 °C. When 2-butyne-1,4-diol was completely dissolved, DBU (19.0 g, 125 mmol, 18.6 mL, 0.20 eq.) was added. *tert*-Butyl acrylate (80.0 g, 624 mmol, 91.0 mL, 1.00 eq.) was mixed with 100 mL 1,4-dioxane and was added *via* dropping funnel over a period of 1 h. The reaction mixture was then stirred at 60 °C for 24 h. The solvent was removed under reduced pressure. The residue was dissolved in a mixture of water and ethyl acetate (1:1 v/v, 600 mL). The aqueous phase was separated and extracted twice with ethyl acetate (2 * 200 mL). The combined organic phases were then washed with distilled water (4 * 500 mL). The organic phase was dried over sodium sulfate and the solvent was removed under reduced pressure. The product tBuA-BYD (tert.-Butyl-3-(4-hydroxybut-2-nyoxy)propanoate) was obtained as yellow-brown liquid. It was redissolved in 200 mL acetonitrile, treated with activated carbon, filtered over a silica plug and isolated as a yellow liquid (108 g, 625 mmol, 81%).

### Example 2:

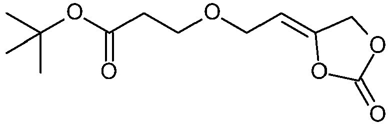

### Synthese of tBuA-exoVC (tert.-Butyl-3-[2-(2-oxo-1,3-dioxolan-4-ylidene)ethoxylpropanoate)

To a solution of tBuA-BYD (tert.-Butyl-3-(4-hydroxybut-2-nyoxy)propanoate) (20.5 g, 27.6 mmol), DavePhos (2-Dicyclohexylphospino- 2'-(N,N-dimethylamino)-biphenyl) (1.94 g, 4.78 mmol) and AgOAc (0.81 g, 4.8 mmol) in DCM (130 ml) was charged to a steel autoclave under atmospheric conditions. The equipment was sealed, and the reaction mixture was pressurized with CO₂ (20 bar) and stirred at room temperature. After 24 hours, CO₂ overpressure was carefully released, and SiOz (30 g) was added. The mixture was filtrated by glass filter and washed with EtOAc. The reaction mixture was washed with water, and the organic phase was dried over anhydrous MgSO₄. The solvent was removed under reduced pressure, and the crude product (tert.-Butyl-3-[2-(2-oxo-1,3-dioxolan-4-ylidene)ethoxy]propanoate) was purified by Kugelrohr distillation (0.001 mbar, 130-145 °C) to give the product (15.1 g, 61%).

### Example 3

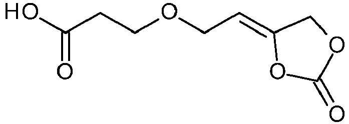

### Svnthese of COOH-exoVC (3-[2-(2-oxo-1,3-dioxolan-4-ylidene)ethoxy]propanoic acid)

To a solution of tBuA-exoVC (tert.-Butyl-3-[2-(2-oxo-1,3-dioxolan-4-ylidene)ethoxy]propanoate) (14.6 g, 53.7 mmol) in acetonitrile (1400 ml) was added 4 M HCl in Dioxane (134 g, 537 mmol) at room temperature, and the mixture was stirred for 1 hour. The solvent was removed under reduced pressure. The crude product was dissolved by MIBK (Methylisobutylketon) (25 ml), and hexane (40 ml) was added to precipitate as oil. The solvent was removed by decantation, and the product (3-[2-(2-oxo-1,3-dioxolan-4-ylidene)ethoxy]propanoic acid) was dried under reduced pressure to give the product (10.7 g, 98%).

### Example 4

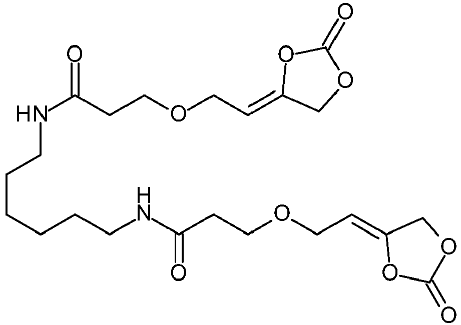

### Svnthese of HDI-bis-exoVC (3-r2-(2-oxo-1,3-dioxolan-4-vlidene)ethoxv1-N-f6-f3-f2-(2-oxo-1,3-dioxolan-4-ylidene)ethoxy]propanoylamino]hexyl]propanamide)

To a solution of COOH-exoVC (3-[2-(2-oxo-1,3-dioxolan-4-ylidene)ethoxy]propanoic acid) (7.60 g, 35.7 mmol) and Magnesium perchlorate (0.19 g, 0.85 mmol) in acetonitrile (20 ml) was added hexamethylene diisocyanate (2.86 g, 17.0 mmol) at room temperature. The reaction mixture was stirred for 22 hours at room temperature. The reaction was quenched by water, and the mixture was extracted with DCM and the organic phase was dried over anhydrous MgSO₄. The solvent was removed under reduced pressure, and the crude product (3-[2-(2-oxo-1,3-dioxolan-4-ylidene)ethoxy]-N-[6-[3-[2-(2-oxo-1,3-dioxolan-4-ylidene)ethoxy]propanoylamino]hexyl]propanamide) was purified by silica gel chromatography to give the product (3.0 g, 31%).

### Example 5

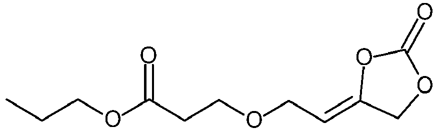

### Svnthese of nPrA-exoVC (propyl 3-r2-(2-oxo-1,3-dioxolan-4-vlidene)ethoxylpropanoate)

To a solution of nPrA-BYD (Propyl-3-(3-hydroxyprop-2-ynoxy)propanoate) (5.52 g, 27.6 mmol), DavePhos (0.56 g, 1.4 mmol) and AgOAc (0.23 g, 1.4 mmol) in DCM (120 ml) was charged to a steel autoclave under atmospheric conditions. The equipment was sealed, and the reaction mixture was pressurized with CO₂ (20 bar) and stirred at room temperature. After 48 hours, CO₂ overpressure was carefully released, and SiO₂ (30 g) was added. The mixture was filtrated by glass filter and washed with DCM and acetonitrile. The reaction mixture was washed with water, and the organic phase was dried over anhydrous MgSO₄. The solvent was removed under reduced pressure, and the crude product (propyl 3-[2-(2-oxo-1,3-dioxolan-4-ylidene)ethoxy]propanoate) was purified by Kugelrohr distillation (0.001 mbar, 124-140 °C) to give the product (1.1 g, 16%).

## Claims

1. Functionalized cyclic carbonates with an exocyclic vinylidene group of formula (I) 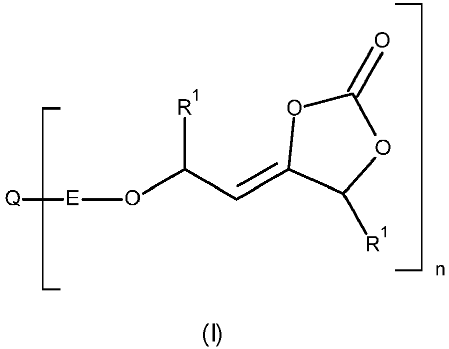 with
n is a natural number selected from the 1 to 10,
R¹ is selected from the group of H and methyl,
E is selected from the group of -CH₂-CH₂-, -CH₂-CH₂-C(O)-, and 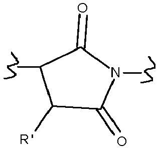 with R' is selected from the group of H and a C₁-C₈ alkyl group, wherein the carbon atom of the terminal -CH₂- or the -CH- group next to the -CHR'-group of E is bonded to the -O- of formula (I)
Q is dependent on n and E
for n=1 and E= -CH₂-CH₂-
Q is selected from the group of -CN and substituted or unsubstituted 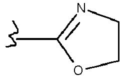 (2-oxazolin) group,
for n=1 and E= -CH₂-CH₂-C(O)-
Q is selected from the group of -O-Y, -NR²-Y and 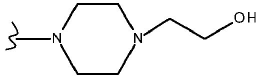 (4-(2-hydroxyethyl)piperazin-1-yl group,
for n=1 and E= 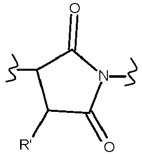
Q is selected from the group of substituted or unsubstituted C₆-C₄₀ aryl groups,
for n=2 and E= -CH₂-CH₂-
Q is selected from the group of a divalent bonded sulfone (-S(O)₂-) group, a divalent substituted or unsubstituted 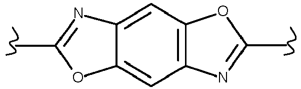 group and a divalent substituted or unsubstituted 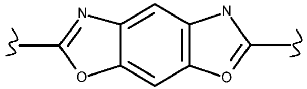 (benzobisoxazol-2,6-diyl) group,
for n=2 and E= -CH₂-CH₂-C(O)-
Q is a divalent -A- group,
for n=2 and E= 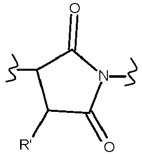
Q is selected from the group of divalently bonded substituted or unsubstituted C₄-C₁₀ alkylen groups, divalently bonded substituted or unsubstituted phenyl divalently bonded substituted or unsubstituted C₁₃-C₄₀ arylalkylenaryl groups and divalently bonded substituted or unsubstituted C₈-C₄₀ alkylenarylalkylen groups,
for n=3 and E= -CH₂-CH₂-C(O)-
Q is selected from the group of a substituted or unsubstituted 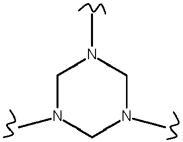 (1,3,5-triazinan) group, 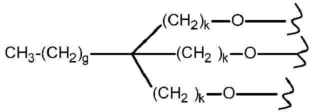 group, 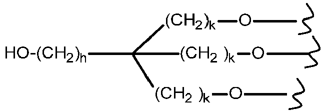 group and 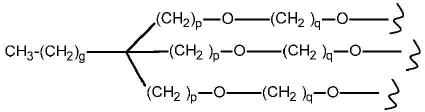 group
wherein
g is a natural number selected from 1 to 33,
k is a natural number selected from 1 to 12,
h is a natural number selected from 1 to 15,
p is a natural number selected from 1 to 3 and
q is a natural number selected from 1 to 5,
for n≥4 and E= -CH₂-CH₂-C(O)-
Q is selected from the group of 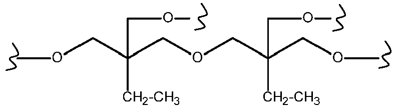 and 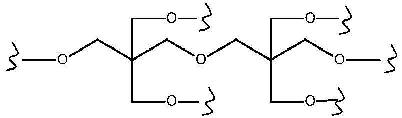 and
with R² selected from the group of H, substituted or unsubstituted C₁-C₄₀ alkyl groups, substituted or unsubstituted C₃-C₄₀ cycloalkyl groups, substituted or unsubstituted saturated or unsaturated heterocyclic groups containing 2 to 20 carbon atoms and one or more heteroatoms selected from the group of N, O, and S as ring members, substituted or unsubstituted C₆-C₄₀ aryl groups, substituted or unsubstituted C₂-C₄₀-alkenyl groups, C₂-C₄₀ alkynyl groups, substituted or unsubstituted C₄-C₄₀ alkylencycloalkyl groups and substituted or unsubstituted C₁-C₄- alkylen groups to be a part of a substituted or unsubstituted saturated or unsaturated heterocyclic group that is built up between R² of the -NR²- group and Y which is not H to form 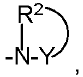
with Y is selected from the group of H, substituted or unsubstituted C₁-C₄₀ alkyl groups, substituted or unsubstituted saturated or unsaturated C₃-C₄₀ cycloalkyl groups, substituted or unsubstituted C₂-C₄₀ (poly)ether groups, substituted or unsubstituted saturated or unsaturated heterocyclic groups containing 2 to 20 carbon atoms and one or more heteroatoms selected from the group of N, O, and S as ring members, substituted or unsubstituted saturated or unsaturated alkylenheterocyclic groups containing 3 to 20 carbon atoms and one or more heteroatoms selected from the group of N, O, and S as ring members, substituted or unsubstituted C₆-C₄₀ aryl groups, substituted or unsubstituted C₂-C₄₀ alkenyl groups, substituted or unsubstituted C₂-C₄₀ alkynyl groups, substituted or unsubstituted saturated or unsaturated C₃-C₄₀ alkylencycloalkyl groups, substituted or unsubstituted C₇-C₄₀ alkylenaryl groups, substituted or unsubstituted α,β- unsaturated carbonyl groups containing C₃-C₄₀ atoms, substituted or unsubstituted C₂-C₅ keto groups, and substituted or unsubstituted C₁-C₄ alkylen group which is part of a substituted or unsubstituted saturated heterocyclic group that is built up between R² of the -NR²- group and Y which is not H to form 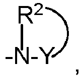
with -A- is one of the following bridging groups:
-X¹-Z-X²-
or 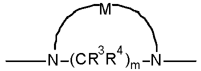
wherein X¹, X² are independently selected from -O-, and -NR²- and
Z is selected from the group of substituted or unsubstituted C₁-C₄₀ alkylen groups, divalently substituted or unsubstituted C₂-C₄₀ (poly)ether groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded substituted or unsubstituted alkylenheterocyclicalkylen groups containing 4 to 20 carbon atoms and one or more heteroatoms selected from the group of N, O, and S as ring members, divalently bonded substituted or unsubstituted C₆-C₄₀ aryl groups, divalently bonded substituted or unsubstituted C₇-C₄₀ cycloalkylalkylencycloalkyl groups, divalently bonded substituted or unsubstituted saturated or unsaturated C₅-C-₄₀ alkylencycloalkylalkylen groups, divalently bonded substituted or unsubstituted C₁₃-C₄₀ arylalkylenaryl groups, divalently bonded substituted or unsubstituted C₈-C₄₀ alkylenarylalkylen groups and divalently bonded substituted or unsubstituted C₁₇-C₃₀ alkylen-bisphenol A -alkylen groups and
R³ and R⁴ are independently selected from the group of H and substituted or unsubstituted C₁-C₄₀ alkyl groups,
m is a natural number selected from the group of 1, 2 or 3 and
M is selected from the group of substituted or unsubstituted C₁-C₃ alkylen groups, divalently bonded -NR⁵-CR³R⁴- groups, divalently bonded -NR⁵-NR⁶- groups, divalently bonded-NR⁵-CR³R⁴-CR³R⁴- groups, divalently bonded -O-CR³R⁴-CR³R⁴- groups, divalently bonded -S-CR³R⁴-CR³R⁴- groups, divalently bonded CR³R⁴-O-CR³R⁴ groups, divalently bonded -CR³R⁴-S-CR³R⁴- groups and divalently bonded -CR³R⁴-NR⁵-CR³R⁴- groups
with R⁵ and R⁶ are independently selected from the group of H, substituted or unsubstituted C₁-C₄₀ alkyl groups, C₆-C₄₀ aryl groups, C₃-C₄₀ cycloalkyl groups and -C(O)CH=CH₂ group.

2. The functionalized cyclic carbonates according to claim 1 wherein E = -CH₂-CH₂- , n is selected from the group of 1 and 2 and Q is selected from the group of -CN, with C₁-C₄ alkylgroups or C₁-C₄ alkylhydroxy groups substituted 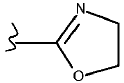 (2-oxazolin) groups, a divalent bonded sulfone (-S(O)₂-), a divalent substituted or unsubstituted 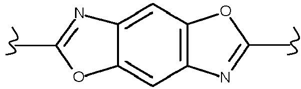 group and a divalent substituted or unsubstituted 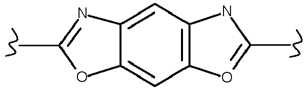 (benzobisoxazol-2,6-diyl) group.

3. The functionalized cyclic carbonates according to claim 1, wherein E = 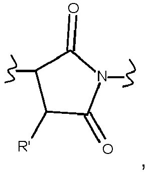 n is selected from the group of 1 and 2 and Q is selected from the group of phenyl, substituted or unsubstituted divalently bonded C₆-C₄₀ aryl groups, divalently bonded substituted or unsubstituted C₁₃-C₄₀ arylalkylenaryl groups, divalently bonded substituted or unsubstituted C₈-C₄₀ alkylenarylalkylen groups.

4. The functionalized cyclic carbonates according to claim 3 wherein Q is selected from the group of phenyl, 1,4-phenylen, 1,3-phenylen, 4-Methyl-1,3-phenylen, 2,2,4-trimethyl-1,6-hexylen, 1,3-Benzenedimethylene (meta-CH₂-Ph-CH₂-), bis(1,4-phenylen)methane (para-CH₂-Ph-CH₂-) and 3,3'-Diethyl-5,5'-dimethyl-4,4'-diphenylenmethane.

5. The functionalized cyclic carbonates according to claim 1, wherein E = -CH₂-CH₂-C(O)-, n is selected from the group of 1 and 2 and Q is selected from the group of -O-Y and -O-Z-O-.

6. The functionalized cyclic carbonates according to claim 1, wherein E = -CH₂-CH₂-C(O)-, n is 1, R¹ is selected from H and methyl and Q is -OH.

7. The functionalized cyclic carbonates according to claim 5, wherein Y is selected from the group of substituted or unsubstituted C₁-C₄₀ alkyl groups, substituted or unsubstituted saturated or unsaturated C₃-C₄₀ cycloalkyl groups, substituted or unsubstituted saturated alkylenheterocyclic groups containing 3 to 20 carbon atoms and one or more heteroatoms selected from the group of N, O, and S as ring members, substituted C₃-C₄₀ unsaturated alkylencycloalkyl groups and Z which is selected from the group of substituted or unsubstituted C₁-C₄₀ alkylen groups, divalently bonded substituted or unsubstituted C₃-C₄₀ cycloalkyl groups, divalently bonded substituted or unsubstituted heterocyclic groups containing 2 to 20 carbon atoms and one or more heteroatoms selected from the group of N, O, and S as ring members, divalently bonded saturated substituted or unsubstituted C₅-C₄₀ alkylencycloalkylalkylen groups and divalently bonded substituted or unsubstituted C₁₇-C₃₀ alkylen-bisphenol A - alkylen groups.

8. The functionalized cyclic carbonates according to claim 1, wherein E = -CH₂-CH₂-C(O)-, n =1 or 2 and Q is selected from the group of -NR²-Y, the 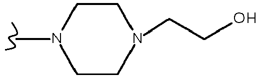 (4-(2-hydroxyethyl)piperazin-1-yl group,-NR²-Z-NR²-, -NR²-Z-O- and 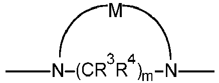 group wherein R², Y, Z, R³, R⁴, M and m are as defined above.

9. The functionalized cyclic carbonates according to claim 8, wherein
R² is selected from the group of H and substituted or unsubstituted C₁-C₄₀ alkyl groups,
Y is selected from the group of substituted or unsubstituted C₁-C₄₀ alkyl groups and substituted or unsubstituted C₆-C₄₀ aryl groups,
Z is selected from the group of substituted or unsubstituted C₁-C₄₀ alkylen groups, divalently bonded substituted or unsubstituted C₆-C₄₀ aryl groups and divalently bonded substituted or unsubstituted alkylenheterocyclicalkylen groups containing 4 to 20 carbon atoms and one or more heteroatoms selected from the group of N, O, and S as ring members,
M is -CH₂-CH₂-,
R³ and R⁴ are H and
m is 2.

10. The functionalized cyclic carbonates according to claim 1, wherein E = -CH₂-CH₂-C(O)-, n is 3 and Q is selected from the group of unsubstituted 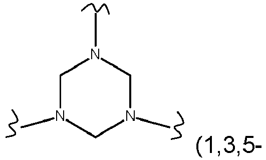 triazinan) group, 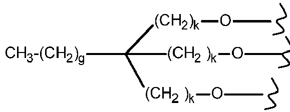 group, 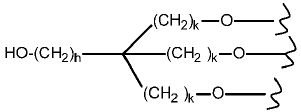 group and 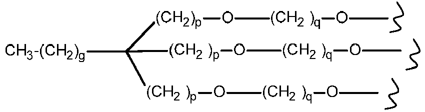 group wherein
g is a natural number selected from 1 to 33,
k is a 1,
h is a natural number selected from 1 to 15,
p is a 1 and
q is a natural number selected from 1 to 5.

11. The functionalized cyclic carbonates according to claim 1, wherein E = -CH₂-CH₂-C(O)-, n ≥ 4 and Q is selected from the group of 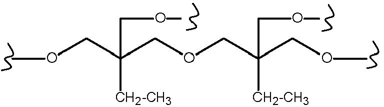 and

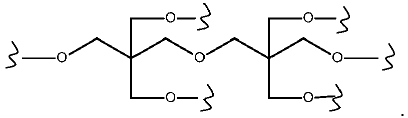

12. A process for the preparation the functionalized cyclic carbonates of formula (I) 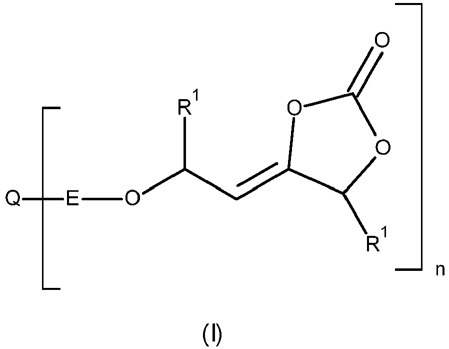 comprising the steps of:
a) reacting an alkynyl ether compound of formula (II) 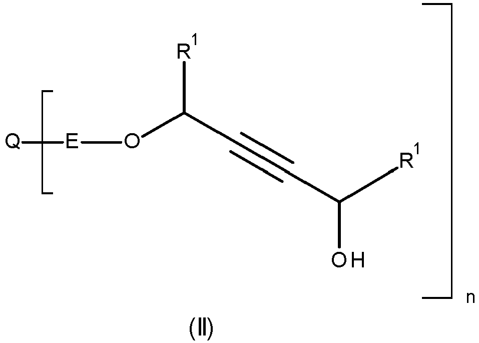 wherein n, R¹, E and Q are as defined above,
with carbon dioxide in the presence of at least one transition metal catalyst TMC1, which comprises Ag as transition metal and at least one bulky ligand selected from the group of compounds of formula (III) and compound of formula (IV) 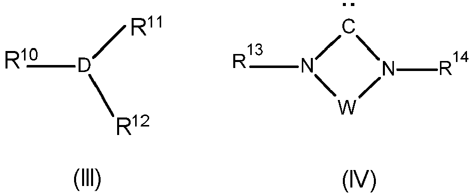 wherein
D is P, As or Sb;
R¹⁰ is selected from the group of a substituted or unsubstituted C₃-C₄₀ cycloalkyl group, a substituted or unsubstituted C₂-C₄₀ heterocycloalkyl group, a substituted or unsubstituted C₆-C₄₀ aryl group, a substituted or unsubstituted C₂-C₄₀ heteroaromatic group, a substituted or unsubstituted C₃-C₄₀ cycloalkoxy group, a substituted or unsubstituted C₂-C₄₀ heterocycloalkoxy group, a substituted or unsubstituted C₆-C₄₀ aryloxy group and a C₂-C₄₀ heteroaryloxy group,
R¹¹ and R¹² are independently of each other selected from the group of substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₂₀ heterocycloalkyl groups, substituted or unsubstituted C₆-C₄₀ aryl groups, substituted or unsubstituted C₂-C₄₀- heteroaromatic groups, substituted or unsubstituted C₆-C₁₀ fluoroaryl groups, substituted or unsubstituted C₂-C₂₀ alkynyl groups and substituted or unsubstituted C₇-C₄₀- alkylenaryl groups,
R¹³ and R¹⁴ are independently of each other selected from the group of substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₄₀ cycloalkoxy group, substituted or unsubstituted C₃-C₂₀ heterocycloalkyl groups, a substituted or unsubstituted C₂-C₄₀ heterocycloalkoxy group, substituted or unsubstituted C₆-C₄₀ aryl groups, substituted or unsubstituted C₆-C₄₀ aryloxy group, substituted or unsubstituted C₂-C₄₀- heteroaromatic groups, substituted or unsubstituted C₆-C₁₀ fluoroaryl groups, substituted or unsubstituted C₂-C₂₀ alkynyl groups and substituted or unsubstituted C₇-C₄₀- alkylenaryl groups, and
W is a divalent bridging group selected from the group of -CR¹⁵=CR¹⁶-, -CR¹⁵=N-, -CR¹⁵R¹⁷-CR¹⁶R¹⁸- and -CR¹⁵R¹⁷-CR¹⁶R¹⁸-CR¹⁹R²⁰-, wherein
R¹⁵, R¹⁶ R¹⁷, R¹⁸, R¹⁹ and R²⁰ are independently of each other selected from the group of hydrogen, substituted or unsubstituted C₁-C₄₀ alkyl groups, substituted or unsubstituted C₃-C₄₀ cycloalkyl group, substituted or unsubstituted C₁-C₁₂ terminal alkoxy groups, substituted or unsubstituted C₃-C₄₀ cycloalkoxy group, substituted or unsubstituted C₃-C₂₀ heterocycloalkyl groups, substituted or unsubstituted C₂-C₄₀ heterocycloalkoxy group, substituted or unsubstituted C₆-C₄₀ aryl groups, substituted or unsubstituted C₆-C₄₀ aryloxy group, substituted or unsubstituted C₂-C₄₀- heteroaromatic groups, substituted or unsubstituted C₆-C₁₀ fluoroaryl groups, substituted or unsubstituted C₂-C₂₀ alkynyl groups, substituted or unsubstituted C₇-C₄₀- alkylenaryl groups and two adjacent groups R¹⁵ and R¹⁶ and/or R¹⁸ and R¹⁹ together with the rest of the group W build up a monocyclic or polycyclic, substituted or unsubstituted, aliphatic or aromatic carbon or heteroatom comprising ring system containing 4 to 40 carbon atoms and wherein the heteroatom comprising ring system comprises one or more heteroatoms selected from the group of Si, Ge, N, P, O and S as ring members.
b) separating the functionalized cyclic carbonates of formula (I) via distillation, extraction, precipitation and/or crystallization
or
for compounds of formula (I) with n = 1, E = -CH₂-CH₂-C(O)- and Q = -OH and compounds of formula (I) with n = 2, E= -CH₂-CH₂-C(O)- and Q= -NH-C₂-C₈-alkyl-NH-, the compounds of formula (I) with n=1, E= -CH₂-CH₂-C(O)- and Q = tert-butyl which are received after step a) and b) wherein an alkynylether compound of formula (II) with n =1, E = -CH₂-CH₂-C(O)- and Q = tert-butyl is used in step a) the resulting compound of formula (I) with n =1, E = -CH₂-CH₂-C(O)- and Q = tert-butyl after step b) will be hydrolysis with an acid selected from the group of anorganic acids and heterogene acid compounds to receive the compound of formula (I) with n = 1, E = -CH₂-CH₂-C(O)- and Q = -OH which will be purified by extraction and crystallization and the resulting product will react with an diisocyanate containing 2 to 13 C- atoms to receive the compound of formula (I) with n = 2, E= -CH₂-CH₂-C(O)- and Q= -NH-C₂-C₈-alkyl-NH-.

13. The process according to claim 12, wherein R¹⁰ of formula (III) and R¹³ of formula (IV) is selected from the group of substituted or unsubstituted C₆-C₄₀ aryl group and a C₂-C₄₀ substituted or unsubstituted heteroaromatic group and R¹⁵, R¹⁶ R¹⁷, R¹⁸, R¹⁹ and R²⁰ in formula (IV) are H.

14. The process according to any of claim 12 to 13, wherein R¹⁰ is substituted in at least one of the two ortho position of the ring cyclus relative to D with a group R^{10a},
wherein R^{10a} is selected form the group of substituted or unsubstituted C₆-C₃₄ aryl group, substituted or unsubstituted C₁-C₁₀ alkoxy group, substituted or unsubstituted C₂-C₁₂ dialkylamino group and where R^{10a} forms with an adjacent group substituting R¹⁰ in the meta position of the ring cyclus together with the atoms connecting them a monocyclic or polycyclic, substituted or unsubstituted aliphatic, aromatic or heteroatom comprising ring system comprising 4 to 40 carbon atoms wherein the heteroatoms are selected from the group of Si, Ge, N, P, O and S.

15. The process according to any of claim 12 to 14, wherein the transition metal catalyst TMC1 is prepared in situ by using a transition metal compound, which does not comprise any bulky ligand, the compound of formula (III) or (IV) as bulky ligand or the protonated form of the compound of formula IV represented by formula (V) 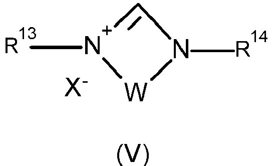 together with a base, wherein R¹⁰, R¹³ and W are defined as above and X- is an anion selected from the group of F-, CI-, Br, I-, PF₆-, NTf₂- ([(CF₃SO₂)₂N]⁻), OTf- (F₃CS(=O)₂-O⁻), MeSO₂O-, MeSO₃O-, OAc, CO₂- and [AlCl₄

16. The process according to any of claim 12 to 15, wherein the transition metal compound is selected from AgOAc, AgF, Ag₂O and Ag₂CO₃.

17. The process according to any of claim 12 to 16, wherein the bulky ligand is a compound of formula (III).

18. The process according to any of claim 12 to 17, wherein the bulky ligand is a compound is selected from a compound A' to P and mixtures thereof

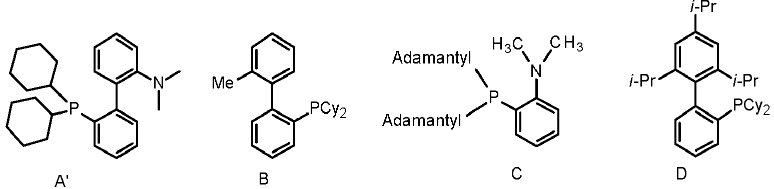

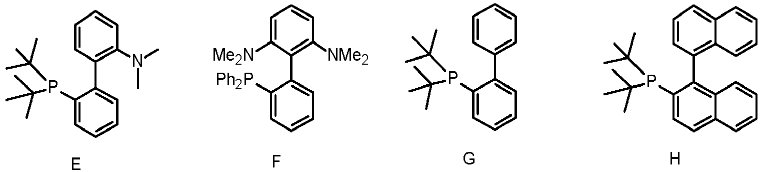

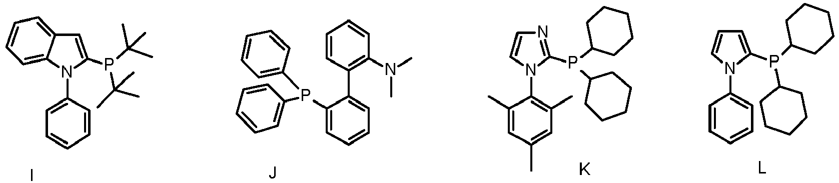

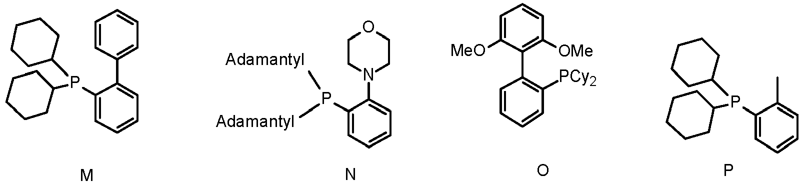

19. The process according to any of claim 12 to 18, wherein the molar ratio of the bulky ligand to the transition metal of transition metal catalyst TMC1 is in the range of 0.4 to 1.2.

20. The process according to any of claim 12 to 19, wherein the amount of transition metal catalyst TMC1 used in step a) based on the amount of alkynyl ether compound of formula (II) is in the range from 0.005 to 5 mol%

21. The process according to any of claim 12 to 20, wherein the pressure during step a) is in the range from 1 to 50 bar.

22. The process according to any of claim 12 to 21, wherein the pressure during step a) is in the range from 0°C to 100°C.

23. The process according to any of claim 12 to 22, wherein the transition metal catalyst TMC1 is recycled to step a) after step b).

24. Use of the cyclic carbonates of any of claim 1 to 10 as precursors of monomers in polymer reactions.
